(19) Europäisches Patentamt
European Patent Office
Office européen des brevets

(11) Publication number: **0 242 973 B1**

(12) # EUROPEAN PATENT SPECIFICATION

(45) Date of publication of patent specification: **17.07.91**

(51) Int. Cl.5: **C07D 403/06**, A61K 31/415

(21) Application number: **87302216.4**

(22) Date of filing: **16.03.87**

(54) **Indole derivatives, method for their preparation and pharmaceutical compositions containing them.**

(30) Priority: **17.03.86 GB 8606579**
**23.07.86 GB 8617995**

(43) Date of publication of application:
**28.10.87 Bulletin 87/44**

(45) Publication of the grant of the patent:
**17.07.91 Bulletin 91/29**

(84) Designated Contracting States:
**BE CH DE FR GB IT LI LU NL SE**

(56) References cited:
**EP-A- 0 003 901**
**DE-A- 3 430 284**

**CHEMICAL ABSTRACTS, vol. 94, no. 11, 16 March 1981, Columbus, OH (US); P.G.BARALDI et al., p. 763, no. 84484n**

(73) Proprietor: **GLAXO GROUP LIMITED**
**Clarges House 6-12 Clarges Street**
**London W1Y 8DH(GB)**

(72) Inventor: **Oxford, Alexander William**
**60 Green Drift**
**Royston Hertfordshire(GB)**
Inventor: **Cavalla, David John**
**20 Thistlewaite Road**
**London, E5 0QQ(GB)**

(74) Representative: **Marchant, James Ian et al**
**Elkington and Fife Beacon House 113 Kingsway**
**London WC2B 6PP(GB)**

**EP 0 242 973 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to heterocyclic compounds, to processes for their preparation, to pharmaceutical compositions containing them and to their medical use. In particular the invention relates to compounds which act upon 5-hydroxytryptamine (5-HT) receptors of the type located on terminals of primary afferent nerves.

Compounds having antagonist activity at 'neuronal' 5-HT receptors of the type located on primary afferent nerves have been described previously.

Thus for example published UK Specification No 2153821 discloses tetrahydrocarbazolones of the general formula

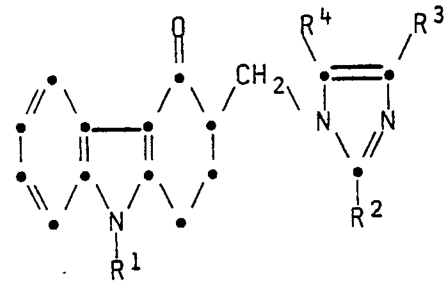

wherein $R^1$ represents a hydrogen atom or a $C_{1-10}$alkyl, $C_{3-7}$cycloalkyl, $C_{3-6}$ alkenyl, phenyl or phenyl $C_{1-3}$alkyl group, and one of the groups represented by $R^2$, $R^3$ and $R^4$ is a hydrogen atom or a $C_{1-6}$alkyl, $C_{3-7}$cycloalkyl, $C_{2-6}$alkenyl or phenyl$C_{1-3}$alkyl group and each of the other two groups, which may be the same or different, represents a hydrogen atom or a $C_{1-6}$ alkyl group.

We have now found a novel group of compounds which differ in structure from those described previously, and which are potent antagonists of the effect of 5-HT at 5-HT 'neuronal' receptors, and possess an advantageous profile of activity.

Thus the present invention provides an indole of the general formula (I):

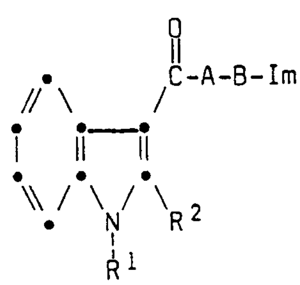

(I)

wherein Im represents an imidazolyl group of formula :

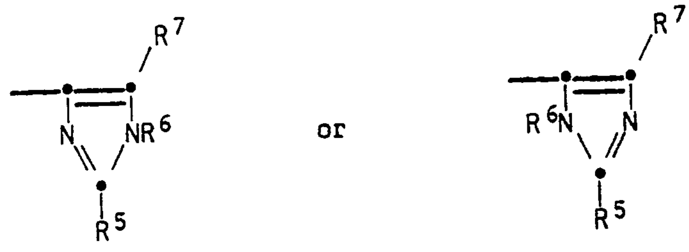

$R^1$ represents a hydrogen atom or a $C_{1-6}$ alkyl, $C_{3-6}$ alkenyl, $C_{3-10}$ alkynyl, $C_{3-7}$ cycloalkyl, $C_{3-7}$ cycloalkyl$C_{1-4}$alkyl, phenyl or phenyl$C_{1-3}$alkyl group;
$R_2$ represents a hydrogen atom or a $C_{1-6}$ alkyl, $C_{3-6}$ alkenyl, $C_{3-7}$ cycloalkyl, phenyl or phenyl$C_{1-3}$alkyl group;
A-B represents the group $R^3R^4C\text{-}CH_2$ or $R^3C=CH$;

$R^3$ and $R^4$, which may be the same or different, each represents a hydrogen atom or a $C_{1-6}$ alkyl group; one of the groups represented by $R^5$,$R^6$ and $R^7$, is a hydrogen atom or a $C_{1-6}$ alkyl, $C_{3-7}$ cycloalkyl, $C_{3-6}$ alkenyl, phenyl or phenyl$C_{1-3}$-alkyl group, and each of the other two groups, which may be the same or different, represents a hydrogen atom or a $C_{1-6}$ alkyl group; and physiologically acceptable salts and solvates thereof.

All optical isomers of compounds or general formula (I) and their mixtures including the racemic mixtures thereof, and all the geometric isomers of compounds of formula (I), are embraced by the invention.

Referring to the general formula (I), the alkyl groups represented by $R^1$,$R^2$,$R^3$,$R^4$,$R^5$,$R^6$ and $R^7$ may be straight chain or branched chain alkyl groups for example, methyl, ethyl, propyl, prop-2-yl, butyl, but-2-yl, pentyl, pent-3-yl or hexyl groups.

A $C_{3-6}$ alkenyl group may be, for example, a propenyl or butenyl group. A $C_{3-10}$ alkynyl group, may be, for example, a prop-2-ynyl or oct-2-ynyl group. It will be appreciated that when $R^1$ represents a $C_{3-6}$alkenyl or $C_{3-10}$ alkynyl group, the double or triple bond respectively may not be adjacent to the nitrogen atom. A phenyl$C_{1-3}$alkyl group may be, for example, a benzyl, phenethyl or 3-phenylpropyl group. A $C_{3-7}$cycloalkyl group, either alone or as part of a $C_{3-7}$cycloalkyl$C_{1-4}$alkyl group, may be, for example, a cyclopropyl, cyclobutyl, cyclopentyl, cyclohexyl or cycloheptyl group.

According to one aspect, the invention provides compounds of formula (I) in which $R^1$ and $R^2$ each independently represent a hydrogen atom or a $C_{1-6}$ alkyl, $C_{3-6}$ alkenyl, $C_{3-7}$ cycloalkyl, phenyl or phenyl$C_{1-3}$alkyl group; one of the groups represented by $R^5$, $R^6$ and $R^7$ is a hydrogen atom or a $C_{1-6}$ alkyl, $C_{3-7}$ cycloalkyl, $C_{3-6}$ alkenyl or phenyl $C_{1-3}$ alkyl group, and each of the other two groups, which may be the same or different, represents a hydrogen atom or a $C_{1-6}$ alkyl group; and A-B, $R^3$ and $R^4$ are as defined in formula (I).

A preferred group of compounds of formula (I) are those in which A-B represents $R^3R^4C\text{-}CH_2$, and $R^1$, $R^2$, $R^3$, $R^4$, $R^5$, $R^6$ and $R^7$ are as defined in formula (I).

A preferred class of compounds of formula (I) are those where $R^1$ represents a hydrogen atom or a $C_{1-3}$ alkyl (eg methyl or isopropyl), $C_{3-4}$ alkenyl (eg prop-2-enyl), $C_{3-4}$ alkynyl (eg prop-2-ynyl), $C_{5-6}$cycloalkyl (eg cyclopentyl), $C_{5-6}$ cycloalkylmethyl (eg cyclopentylmethyl) or benzyl group. Most preferably $R^1$ represents a hydrogen atom or a $C_{1-3}$ alkyl, $C_{3-4}$alkenyl,$_{3-4}$alkynyl, $C_{5-6}$ cycloalkyl or benzyl group.

Another preferred class of compounds of formula (I) are those wherein $R^2$ represents a phenyl group, or more preferably, a hydrogen atom or a $C_{1-3}$ alkyl (eg methyl) group.

Another preferred class of compounds of formula (I) are those wherein A-B represents CH=CH or $R^3R^4C\text{-}CH_2$ where $R^3$ and $R^4$ each independently represent a hydrogen atom or a $C_{1-3}$ alkyl (eg methyl) group. A particularly preferred class of compounds are those in which A-B represents $R^3R^4C\text{-}CH_2$ and $R^3$ and $R^4$ each independently represent a hydrogen atom or a methyl group.

A further preferred class of compounds of formula (I) are those wherein $R^5$ represents a $C_{1-3}$ alkyl (eg methyl) group or, more preferably, a hydrogen atom. $R^6$ preferably represents a $C_{1-3}$ alkyl (eg methyl), $C_{3-4}$ alkenyl (eg prop-2-enyl), benzyl or, more preferably, a hydrogen atom. $R^7$ preferably represents a hydrogen atom or a $C_{1-3}$ alkyl (eg methyl or n-propyl) group. When $R^5$ and $R^6$ represent hydrogen atoms, $R^7$ is preferably a $C_{1-6}$ alkyl, $C_{3-7}$ cycloalkyl, $C_{3-6}$ alkenyl, phenyl or phenyl$C_{1-3}$ alkyl group, more particularly $C_{1-3}$ alkyl (eg methyl).

A preferred group of compounds of formula (I) are those in which $R^1$ represents a hydrogen atom or a $C_{1-3}$ alkyl, $C_{3-4}$ alkenyl, $C_{3-4}$ alkynyl, $C_{5-6}$ cycloalkyl or benzyl group; $R^2$ represents a hydrogen atom or a $C_{1-3}$ alkyl group; A-B represents CH=CH or $R^3R^4C\text{-}CH_2$ where $R^3$ and $R^4$ each independently represent a hydrogen atom or a $C_{1-3}$ alkyl group; and $R^5$, $R^6$ and $R^7$ each independently represent a hydrogen atom or a $C_{1-3}$ alkyl group.

A particularly preferred group of compounds of formula (I) are those wherein $R^1$ represents a hydrogen atom or a methyl, prop-2-enyl or cyclopentyl group; $R^2$ represents a hydrogen atom or a methyl group; A-B represents $CR^3R^4\text{-}CH_2$ where $R^3$ and $R^4$ each independently represent a hydrogen atom or a methyl group; $R^5$ and $R^6$ each represent a hydrogen atom; and $R^7$ represents a hydrogen atom or a $C_{1-3}$ alkyl group, most preferably methyl.

Particularly preferred compounds according to the invention are:
3-(5-methyl-1H-imidazol-4-yl)-1-(1-methyl-1H-indol-3-yl)-1-propanone;
2-methyl-3-(5-methyl-1H-imidazol-4-yl)-1-(1-methyl-1H-indol-3-yl)-1-propanone;
3-(5-methyl-1H-imidazol-4-yl)-1-(1,2-dimethyl-1H-indol-3-yl)-1-propanone;
1-(1-methyl-1H-indol-3-yl)-3-(5-propyl-1H-imidazol-4-yl)-1-propanone;
2,2-dimethyl-3-(5-methyl-1H-imidazol-4-yl)-1-(1-methyl-1H-indol-3-yl)-1-propanone;
and their physiologically acceptable salts and solvates.

Suitable physiologically acceptable salts of the indoles of general formula (I) include acid addition salts

formed with organic or inorganic acids for example, hydrochlorides, hydrobromides, sulphates, phosphates, citrates, succinates, tartrates, fumarates and maleates. The solvates may, for example, be hydrates.

Compounds of the invention are potent and selective antagonists of 5-HT-induced depolarisation of the rat isolated vagus nerve preparation and thus act as potent and selective antagonists of the 'neuronal' 5-HT receptor type located on primary afferent nerves. Receptors of this type are now designated as 5-$HT_3$ receptors. Such receptors are also believed to be present in the central nervous system. 5-HT occurs widely in the neuronal pathways in the central nervous system and disturbance of these 5-HT containing pathways is known to alter aspects of behaviour such as mood, psychomotor activity, appetite and memory.

Compounds of formula (I), which antagonise the effect of 5-HT at 5-$HT_3$ receptors, are useful in the treatment of conditions such as psychotic disorders (e.g. schizophrenia and mania); anxiety; and nausea and vomiting. Compounds of formula (I) are also useful in the treatment of gastric stasis; symptoms of gastrointestinal dysfunction such as occur with dyspepsia, peptic ulcer, reflux oesophagitis, flatulence and irritable bowel syndrome; migraine; and pain.

Unlike existing drug treatments for those conditions, the compounds of the invention, because of their high selectivity for 5-$HT_3$ receptors, would not be expected to produce undesirable side effects. Thus, for example, neuroleptic drugs may exhibit extrapyramidal effects, such as tardive dyskinesia, and benzodiazepines may cause dependence.

Compounds of formula (I) and physiologically acceptable salts and solvates thereof are useful in the treatment of a human or animal subject suffering from a psychotic disorder such as schizophrenia or mania; or from anxiety; nausea or vomiting; gastric stasis; symptoms of gastrointestinal dysfunction such as dyspepsia, reflux oesophagitis, peptic ulcer, flatulence and irritable bowel syndrome; migraine; or pain.

Accordingly, the invention also provides a pharmaceutical composition which comprises at least one compound selected from indole derivatives of the general formula (I), and their physiologically acceptable salts and solvates e.g. hydrates, adapted for use in human or veterinary medicine, and formulated for administration by any convenient route.

Such compositions may be formulated in conventional manner using one or more physiologically acceptable carriers or excipients.

Thus the compounds according to the invention may be formulated for oral, buccal, parenteral or rectal administration or in a form suitable for administration by inhalation or insufflation (either through the mouth or nose).

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxylpropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. almond oil, oily esters, ethyl alcohol or fractionated vegetable oils); and preservatives (e.g. methyl or propyl-p-hydroxybenzoates or sorbic acid). The preparations may also contain buffer salts, flavouring, colouring and sweetening agents as appropriate.

Preparations for oral administration may be suitably formulated to give controlled release of the active compound.

For buccal administration the compositions may take the form of tablets or lozenges formulated in conventional manner.

The compounds of the invention may be formulated for parenteral administration by injection. Formulations for injection may be presented in unit dosage form e.g. in ampoules or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

The compounds of the invention may also be formulated in rectal compositions such as suppositories or retention enemas, e.g. containing conventional suppository bases such as cocoa butter or other glycerides.

In addition to the formulations described previously, the compounds of the invention may also be formulated as depot preparations. Such long acting formulations may be administered by implantation (for

example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For administration by inhalation the compounds according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane, carbon dioxide or other suitable gas. In the gas of a pressurised aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

A proposed dose of the compounds of the invention for administration to man (of approximately 70kg body weight) is 0.001 to 100mg, preferably 0.01 to 50mg of the active ingredient per unit dose which could be administered, for example, 1 to 4 times per day. It will be appreciated that it may be necessary to make routine variations to the dosage, depending on the age and condition of the patient. The dosage will also depend on the route of administration.

According to another aspect of the invention, compounds of general formula (I) and physiologically acceptable salts or solvates or physiologically acceptable equivalents thereof may be prepared by the general methods outlined hereinafter. In the following description, the groups $R^1$ to $R^7$, A,B and Im are as defined for compounds of general formula (I) unless otherwise stated.

According to a first general process (A), a compound of general formula (I), wherein A-B represents the group $R^3R^4C\text{-}CH_2$, or a physiologically acceptable salt or solvate thereof, may be prepared by reacting a compound of formula (II):

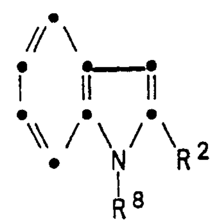

(II)

(wherein $R^8$ represents a group $R^1$ as previously defined or a group MgHal, where Hal is a halide ion eg a bromide or iodide ion) or a protected derivative thereof (eg an N-phenylsulphonyl derivative), with an acylating reagent derived from an acid of general formula (III) :

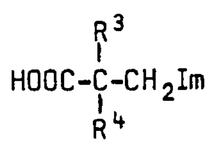

(III)

or a salt or protected derivative thereof, followed where necessary by removal of any protecting groups.

Suitable acylating reagents include acid halides (e.g. acid chlorides), anhydrides (e.g. symmetrical anhydrides or mixed anhydrides formed for example with pivaloyl chloride), amides, and nitriles.

When the group $R^8$ represents $R^1$, an indole of formula (II) may be condensed with an acid halide (e.g. an acid chloride) or an anhydride (e.g. pivalic anhydride) derivative of the acid (III) under Friedel-Crafts conditions. In this particular embodiment of general process (A), $R^1$ preferably represents an acyl group. Thus, the reaction is desirably conducted in the presence of a Lewis acid, such as stannic chloride or aluminium chloride. The reaction may conveniently be effected in an organic solvent such as a halogenated hydrocarbon (e.g. dichloromethane or dichloroethane) or carbon disulphide, and at a temperature in the range -5 to +85° C.

When employing an indole of formula (II) in which $R^8$ represents MgHal, the acylation reaction with an acid halide or anhydride may be effected in a non-polar organic solvent such as an ether (e.g. diethyl ether) or an aromatic hydrocarbon (e.g. toluene) or mixtures thereof, at a temperature in the range -5 to +80° C.

Indoles of formula (II) may also be acylated according to the Vilsmeier-Haack reaction, using a tertiary amide derivative of an acid of formula (III), such as the corresponding N,N-dimethyl propanamide

compound in the presence of a phosphoryl halide such as phosphorous oxychloride. This reaction may be effected in the presence or absence of a solvent. Solvents which may conveniently be employed include halogenated hydrocarbons such as 1,2-dichloroethane. The reaction temperature may be in the range 20 to 100 °C. In this embodiment of general process (A), $R^8$ is preferably a group $R^1$.

Indoles of formula (I) wherein $R^2$ is other than hydrogen may be prepared according to general process (A) by reaction with a nitrile corresponding to the acid of formula (III), in the presence of hydrogen chloride. If an indole of formula (II) in which $R^8$ represents MgHal is employed, this may first be reacted with the nitrile to form an imine, which may subsequently be converted into the corresponding ketone by hydrolysis with an acid.

Acylating reagents corresponding to the acids of formula (III) may be prepared by conventional methods. Thus, an acid halide may be prepared by reacting an acid (III) or a salt thereof with a halogenating agent (e.g. thionyl chloride or phosphorus pentachloride).

Anhydrides may be prepared by reacting an acid (III) with an appropriate acid halide in the presence of a base, or alternatively by reacting an acid halide corresponding to the compound of formula (III) with an acid, in the presence of a base.

An amide may be prepared by reaction of the corresponding acid chloride with the appropriate amine (e.g. dimethylamine). Alternatively an amide corresponding to an acid (III) may be prepared by catalytic hydrogenation of the corresponding propenamide compound, which may itself be obtained from the appropriate propenoic acid via an acid halide derivative.

A nitrile corresponding to an acid of formula (III) may be prepared by dehydration of the corresponding unsubstituted amide derivative, using a conventional dehydrating agent such as phosphorus pentoxide, phosphorous oxychloride or phosphorus pentachloride.

Acids of general formula (III) may themselves be prepared for example from the corresponding esters by acid- or base-catalysed hydrolysis.

The esters may be prepared for example by reacting an aldehyde of formula (IV):

OHC-Im (IV)

or a protected derivative thereof with a phosphonate of formula (V):

$$(R^{10}O)_2\overset{O}{\overset{\|}{P}}CHR^3CO_2R^9 \qquad (V)$$

wherein $R^9$ represents a group such as an alkyl (e.g. methyl or ethyl), aryl (e.g. phenyl) or aralkyl (e.g. benzyl) group and $R^{10}$ represents an alkyl (e.g. ethyl) or aryl (e.g. phenyl) group, which has been pre-treated with a base such as an alkali metal hydride (eg sodium hydride) in an inert organic solvent such as an ether (eg tetrahydrofuran) or a substituted amide (eg dimethylformamide), to give a compound of formula (VI):

$$R^9O_2C\overset{R^3}{\overset{|}{C}}{=}CH\text{-}Im \qquad (VI)$$

followed by catalytic hydrogenation of the double bond, using for example palladium on charcoal as the catalyst. Where it is desired to produce an ester corresponding to the acid (III) in which $R^3$ and $R^4$ both represent alkyl group, the hydrogenation may be followed by alkylation to introduce the desired $R^4$ group and where appropriate, $R^3$.

Compounds of formula (IV) can be prepared, for example, by oxidation of the corresponding methanol-substituted imidazole compound, or a protected derivative thereof, with an oxidising agent such as manganese dioxide.

According to another general process (B) a compound of general formula (I), wherein A-B represents the group $R^3C=CH$, or a physiologically acceptable salt or solvate thereof, may be prepared by condensing a compound of formula (VII) :

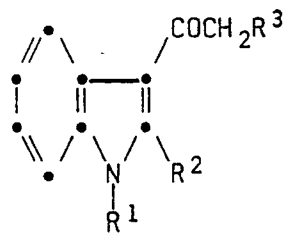

(VII)

or a protected derivative thereof, with a compound of formula (IV), or a protected derivative thereof, in the presence of a base, followed where necessary by removal of any protecting groups. The reaction may conveniently be effected using an alkali metal hydroxide (eg sodium or potassium hydroxide) in an alcohol (eg ethanol or t-butanol) or water, or mixtures thereof, or using an alkali metal alkoxide (eg sodium ethoxide or potassium t-butoxide) in the corresponding alcohol (eg ethanol or t-butanol) or in an inert solvent such as an ether (eg tetrahydrofuran), and at a temperature of 0 to 100° C.

Compounds of formula (VII) may be prepared by treating an indole of formula (II) with an acylating derivative of an acid of formula (VIII):

$HOOCCH_2R^3$ (VIII)

under conditions analogous to those described for process (A) above.

According to another general process (C), a compound of general formula (I), or a salt or protected derivative thereof, may be converted into another compound of formula (I) using conventional techniques. Such conventional techniques include hydrogenation and alkylation.

Thus, for example, compounds of formula (I) in which A-B represents the group $R^3CH\text{-}CH_2$ may be prepared by hydrogenating the corresponding compounds of formula (I) in which A-B represents the group $R^3C=CH$. Hydrogenation may also be used to convert an alkenyl or an alkynyl substituent into an alkyl substituent, or an alkynyl into an alkenyl substituent.

Hydrogenation according to general process (C) may be effected using conventional procedures, for example using hydrogen in the presence of a noble catalyst (e.g. palladium, Raney nickel, platinum or rhodium). The catalyst may be supported on, for example, charcoal, or alternatively a homogeneous catalyst such as tris(triphenylphosphine)rhodium chloride may be used. The hydrogenation will generally be effected in a solvent such as an alcohol (e.g. ethanol), an ether (e.g. dioxan), or an ester (e.g. ethyl acetate), and at a temperature in the range -20 to +100° C, preferably 0 to 50° C.

Alkylation according to general process (C) may be effected for example on a compound of formula (I) where one or more of $R^1$, $R^3$, $R_4$, $R^5$ and $R^6$ represent a hydrogen atom.

The term 'alkylation' also includes the introduction of other groups such as cycloalkyl or alkenyl groups. Thus, for example, a compound of formula (I) in which $R^1$ represents a hydrogen atom may be converted into the corresponding compound in which $R^1$ represents a $C_{1\text{-}6}$ alkyl, $C_{3\text{-}7}$ cycloalkyl, $C_{3\text{-}6}$ alkenyl, $C_{3\text{-}10}$ alkynyl,
$C_{3-7}$cycloalkyl$C_{1-4}$alkyl or phenyl$C_{1-3}$alkyl group, or a compound in which at least one of $R^3$ and $R^4$ represents a hydrogen atom may have the hydrogen atom(s) replaced by a $C_{1-6}$alkyl group. Similarly, compounds of formula (I) in which $R^6$ represents a hydrogen atom may be 'alkylated' to give compounds of formula (I) in which $R^6$ represents a $C_{1-6}$ alkyl, $C_{3-7}$cycloalkyl, $C_{3-6}$ alkenyl or phenyl $C_{1-3}$ alkyl group.

The above alkylation reactions may be effected using the appropriate alkylating agent selected from compounds of formula $R^{11}Z$ where $R^{11}$ represents a $C_{1-6}$ alkyl, $C_{3-7}$ cycloalkyl, $C_{3-6}$ alkenyl, $C_{3-10}$ alkynyl, $C_{3-7}$ cycloalkyl$C_{1-4}$alkyl or phenyl$C_{1-3}$alkyl group, and Z represents a leaving atom or group such as a halogen atom (e.g. chlorine or bromine), or an acyloxy group (e.g. acetoxy, trifluoromethanesulphonyloxy, p-toluenesulphonyloxy or methanesulphonyloxy); or a sulphate of formula $(R^{11})_2SO_4$.

The alkylation reaction is conveniently carried out in an inert organic solvent such as a substituted amide (eg dimethylformamide), an ether (eg tetrahydrofuran) or an aromatic hydrocarbon (eg toluene), preferably in the presence of a base. Suitable bases include, for example, alkali metal hydrides (eg sodium hydride), alkali metal amides (eg sodium amide or lithium diisopropylamide), alkali metal carbonates (eg sodium carbonate) or an alkali metal alkoxide (eg sodium or potassium methoxide, ethoxide or t-butoxide). The reaction may conveniently be effected a temperature in the range -70 to +100° C, preferably 0 to 50° C.

According to another general process (D) a compound of general formula (I) wherein A-B represents the

group $R^3R^4C\text{-}CH_2$, or a physiologically acceptable salt or solvate thereof, may be prepared by oxidising a compound of formula (IX):

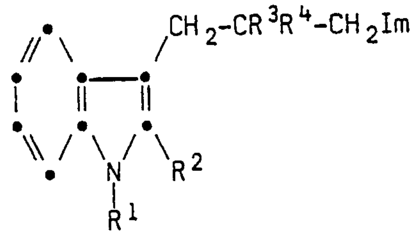

(IX)

or a protected derivative thereof, followed where necessary by removal of any protecting groups. The oxidation process may be effected using conventional methods and the reagents and reaction conditions should be chosen such that they do not cause oxidation of the indole group. Thus, the oxidation process is preferably effected using a mild oxidising agent.

Suitable oxidising agents include quinones in the presence of water (eg 2,3-dichloro-5,6-dicyano-1,4-benzoquinone or 2,3,5,6-tetrachloro-1,4-benzoquinone), selenium dioxide, a cerium (IV) oxidising agent (eg ceric ammonium nitrate), a chromium (VI) oxidising agent (eg a solution of chromic acid in acetone, for example Jones' reagent) or chromium trioxide in pyridine.

Suitable solvents may be selected from ketones (eg acetone or butanone), ethers (eg tetrahydrofuran or dioxan), amides (eg dimethylformamide), alcohols (eg methanol), hydrocarbons (eg toluene), halogenated hydrocarbons (eg dichloromethane) and water or mixtures thereof.

The process is conveniently at a temperature of -70 to +50 °C. It will be understood that the choice of oxidising agent will effect the preferred reaction temperature and solvent.

Compounds of formula (IX) may be prepared by reduction of a compound of formula (I), or a protected derivative thereof, using a suitable reducing agent. Suitable reducing agents include diisobutylaluminium hydride, in a suitable solvent such as an ether (eg tetrahydrofuran), a hydrocarbon (eg hexane or toluene) or a halogenated hydrocarbon (eg dichloromethane) at a temperature of $^-80$ to +25 °C, and lithium aluminium hydride in a suitable solvent such as an ether (eg tetrahydrofuran or ether) at a temperature of $^-20$ to + 50 °C.

According to another general process (E), a compound of general formula (I), wherein A-B represents the group $R^3R^4C\text{-}CH_2$, or a physiologically acceptable salt or solvate thereof, may be prepared by reacting a compound of formula (X) :

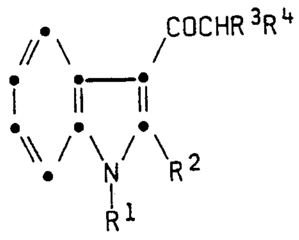

(X)

or a protected derivative thereof, with a compound of formula (XI) :

$LCH_2\text{-}Im$ (XI)

wherein L represents a leaving atom or group, such as a halogen atom (eg chlorine or bromine), or an acyloxy group (eg acetoxy, trifluoromethanesulphonyloxy, p-toluenesulphonyloxy or methanesulphonyloxy), or a protected derivative thereof, in the presence of a base, followed where necessary by removal of any protecting groups.

Suitable bases include alkali metal hydrides (eg sodium or potassium hydride), alkali metal alkoxides (eg potassium-t-butoxide) or lithium diisopropylamide. The reaction may conveniently be carried out in an inert solvent such as an ether (eg tetrahydrofuran), a substituted amide (eg dimethylformamide), or an aromatic hydrocarbon (eg toluene) and at a temperature of $^-70$ to +50 °C.

It should be appreciated that in the above transformations it may be necessary or desirable to protect

any sensitive groups in the molecule of the compound in question to avoid undesirable side reaction. For example, it may be necessary to protect the keto group, for example, as a ketal or a thioketal. It may also be necessary to protect the indole and/or imidazole nitrogen atoms, for example with an arylmethyl (e.g. benzyl or trityl), alkyl (e.g. t-butyl), alkoxymethyl (e.g. methoxymethyl), acyl (eg benzyloxycarbonyl) or a sulphonyl (eg N,N-dimethylaminosulphonyl or p-toluenesulphonyl) group.

Thus according to another general process (F), a compound of formula (I) may be prepared by the removal of any protecting groups from a protected form of a compound of formula (I). Deprotection may be effected using conventional techniques such as those described in 'Protective Groups in Organic Synthesis' by Theodora W. Greene (John Wiley and Sons, 1981).

For example a ketal such as an alkyleneketal group may be removed by treatment with a mineral acid such as hydrochloric acid. A thioketal group may be cleaved by treatment with a mercuric salt, (e.g. mercuric chloride), in a suitable solvent, such as ethanol. An arylmethyl N-protecting group may be cleaved by hydrogenolysis in the presence of a catalyst (e.g. palladium on charcoal) and a trityl group may also be cleaved by acid hydrolysis )e.g. using dilute hydrochloric or acetic acid). An alkoxyalkyl group may be removed using a Lewis acid such as boron tribromide. An acyl group may be removed by hydrogenation (eg with sodium in liquid ammonia) or under acidic conditions (eg using hydrogen bromide or trifluoroacetic acid). A sulphonyl group may be removed by alkaline hydrolysis.

Where it is desired to isolate a compound of the invention as a salt, for example a physiologically acceptable salt, this may be achieved by treating the free base of general formula (I) with an appropriate acid, preferably with an equivalent amount, in a suitable solvent such as an alcohol (e.g. ethanol or methanol), an ester (eg ethyl acetate) or an ether (eg tetrahydrofuran).

Physiologically acceptable equivalents of a compound of formula (I) may be prepared according to conventional methods. Thus, for example, an N-acyl derivative may be prepared using conventional acylation techniques.

Individual enantiomers of the compounds of the invention may be obtained by resolution of a mixture of enantiomers (e.g a racemic mixture) using conventional means, such as an optically active resolving acid; see for example 'Stereochemistry of Carbon Compounds' by E.L.Eliel (McGraw Hill 1962) and 'Tables of Resolving Agents' by S. H. Wilen.

The methods indicated above for preparing the compounds of the invention can be used as the main step in the preparative sequence. The same general methods can be used for the introduction of the desired groups at an intermediate stage in the stepwise formation of the required compound, and it will be appreciated that these general methods can be combined in different way in such multi-stage processes. The sequence of the reaction in multi-stage processes should of course be chosen so that the reaction conditions used do not affect groups in the molecule which are desired in the final product.

The invention is further illustrated by the following Examples. All temperatures are in °C. Thin layer chromatography (t.l.c) was carried out over silica, and flash column chromatography (FCC) and short-path column chromatography (SPCC) on silica (Merck 9385 and Merck 7747 respectively). Solvent System A as used for chromatography denotes dichloromethane:ethanol:0.88 ammonia solution. Organic extracts were dried over sodium sulphate or magnesium sulphate. The following abbreviations are used: DMF - dimethylformamide, THF - tetrahydrofuran, IMS - industrial methylated spirits.

Intermediate 1

**(E)-N,N-Dimethyl-3-(1H-imidazol-4-yl)-2-propenamide**

A mixture of urocanic acid (1.38g) and phosphorus pentachloride (2.08g) was heated under nitrogen at 100-110°C for 1.5h, then at 190° for 1 h. The mixture was cooled, washed twice with dichloromethane (50mℓ) and filtered. The solid was treated with a solution of dimethylamine in I.M.S. (33% w/v; 50 mℓ) and stirred at room temperature for 2h. The mixture was evaporated in vacuo, dissolved in ethanol (25mℓ) and purified by FCC, eluting with System A (89:10:1), to give a gum. This was triturated with dry ether (30mℓ), to give the title compound (0.95g) as a solid, m.p. 142-144° (decomp.).

Intermediate 2

**N,N-Dimethyl-3-(1H-imidazol-4-yl)-1-propanamide hydrochloride**

A suspension of 10% palladium oxide on carbon (0.18g) in ethanol (20mℓ) was stirred under hydrogen for 30min. A solution of (E)-N,N-dimethyl-3-(1H-imidazol-4-yl)-2-propenamide (1.0g) in ethanol (40mℓ) was

added and stirring was continued overnight (18h). The suspension was filtered through Hyflo and evaporated in vacuo to give an oil (1.12g), which was dissolved in ethanol (15mℓ) and acidified with ethereal hydrogen chloride. The resulting precipitate was filtered off, washed with dry ether (ca. 100 mℓ) and dried (20mmHg, 100°, 3h) to give the crystalline title compound (1.11g), m.p. 171-173°.

Intermediate 3

**5-Methyl-1-(triphenylmethyl)-1H-imidazole-4-methanol**

A solution of triphenylchloromethane (13.1g) in dry DMF (80ml) was added dropwise over 0.5h to a stirred solution of 4-methyl-5-imidazolemethanol hydrochloride (7.0g) and triethylamine (9.52g) in dry DMF (75ml) at room temperature under nitrogen, and stirring was continued for 2.5h. The suspension was poured onto ice (600ml), stirred for 0.5h and filtered to give a solid (12.0g). This solid was triturated twice with acetone (2 x 250ml) to give the title compound (8.4g) as a white solid, t.l.c. (System A 94.5:5:0.5), Rf 0.19.

Intermediate 4

**5-Methyl-1-(triphenylmethyl)-1H-imidazole-4-carboxaldehyde**

A mixture of 5-methyl-1-(triphenylmethyl)-1H-imidazole -4-methanol (4.0g), manganese dioxide (activated) (40g) and dioxan (225ml) was stirred at room temperature overnight. The suspension was filtered through Hyflo, the solid washed with hot chloroform (1ℓ) and the combined filtrates evaporated in vacuo to leave an off-white solid (4.0g). This solid was purified by FCC eluting with chloroform to give an off-white solid which was triturated with hexane (ca. 50 ml) to give the title compound(2.99g) as a white crystalline solid, m.p. 184-188° (decomp.).

Intermediate 5

**(E)-N,N-Dimethyl-3-[5-methyl-1-(triphenylmethyl)-1H-imidazol-4-yl]-2-propenamide**

A solution of dimethyl [2-(dimethylamino)-2-oxoethyl]phosphonate (4.88g) in dry THF (25mℓ was added dropwise with stirring to a suspension of sodium hydride (1.54g of a 78% dispersion in oil pre-washed with hexane (2x40mℓ) in dry THF (25mℓ) under nitrogen at -10°. The suspension was stirred at room temperature for 1h, cooled to -10° and 5-methyl-1-(triphenylmethyl)-1H-imidazole-4-carboxaldehyde (6.51g) in dry THF (50mℓ) was added. The mixture was stirred at room temperature for 64h, poured into 8% aqueous sodium bicarbonate (20mℓ) and extracted with ethyl acetate (3x200mℓ). The combined, dried organic extracts were evaporated to give a yellow semi-solid (8.0g). This was purified by FCC eluting with dichloromethane:ethanol (95:5) to give on trituration with hexane the title compound (6.4g) as a white solid, m.p. 195-197°.

Intermediate 6

**N,N-Dimethyl-3-(5-methyl-1H-imidazol-4-yl)propanamide**

A solution of (E)-N,N-dimethyl-3-[5-methyl-1-(triphenylmethyl)-1Himidazol-4-yl]-2-propenamide (6.1g) in absolute ethanol (100mℓ) was added to a suspension of pre-reduced 10% palladium oxide on charcoal (2.0g) in absolute ethanol (50mℓ). The mixture was stirred in a hydrogen atmosphere for 72h then filtered (Hyflo) and evaporated to give a grey oily solid (ca.6g). Column chromatography on silica gel (Merck 7734), made up in EtOAc:MeOH:$Et_3N$ (80:19:1) eluting with ethyl acetate:methanol (4:1) gave the title compound - (2.52g) as a white solid, m.p. 62-64°.

Intermediate 7

**N,N-Dimethyl-3-(5-methyl-1H-imidazol-4-yl)propanamide dihydrochloride**

A solution of (E)-N,N-dimethyl-3-[5-methyl-1-(triphenylmethyl)-1H-imidazol-4-yl]-2-propenamide (6.0g) in ethanol (100mℓ) was hydrogenated at room temperature and pressure over 10% palladium oxide on carbon (dry, 2.0g, pre-reduced in ethanol (25mℓ) for 48h. The catalyst was filtered off (Hyflo), replaced with fresh

palladium oxide (2.0g) and hydrogenation continued for 72h. The catalyst was filtered off (Hyflo) and the filtrate evaporated *in vacuo* to yield a yellow oil. This was dissolved in ethanol (15mℓ) and acidified with ethanolic hydrogen chloride (to pH1). Dry ether (500mℓ) was added and the resulting precipitate was filtered off to give the *title compound* (1.72g) as a white powder, m.p. 142-144°.

Intermediate 8

**4-Iodo-2-methyl-1-(4-methylbenzenesulphonyl)-1*H*-imidazole**

Sodium hydrogen carbonate (9g) was added to a stirred solution of 4-iodo-2-methyl-1*H*-imidazole (9.0g) in acetonitrile (125mℓ) followed by a solution of p-toluenesulphonyl chloride (8.3g) in acetonitrile (50mℓ). The resulting mixture was maintained at ca. 40° for 5h. The reaction mixture was filtered, concentrated *in vacuo* to ca. 30 mℓ and n-hexane (ca 350mℓ) was added, precipitating the *title compound* (7.8g) as crystals, m.p. 113-114°. Concentration of the mother liquors (to *ca.* 50 mℓ) afforded further *title compound* (6.1g), m.p. 112-114°.

Intermediate 9

**(*E*)-*N*,*N*-Dimethyl-3-[2-methyl-1-(4-methylbenzenesulphonyl)-1*H*-imidazol -4-yl-2- propenamide**

A mixture of 4-iodo-2-methyl-1-(4-methylbenzenesulphonyl)-1H-imidazole (3.62g), *N*,*N*-dimethylacrylamide (1.05g), palladium (II) acetate (125mg) and triethylamine (2mℓ) in acetonitrile (3mℓ) was heated at 120° under nitrogen for 2.5h. The cooled mixture was partitioned between chloroform (3x50mℓ) and 2N sodium carbonate (75mℓ), and the combined organic layers were dried and evaporated *in vacuo* to leave a solid (ca. 3.0g). This solid was purified by FCC eluting with chloroform to give the crystalline *title compound* (1.55g), m.p. 162-163°.

Intermediate 10

**(*E*)-*N*,*N*-Dimethyl-3-(2-methyl-1*H*-imidazol-4-yl)-2-propenamide**

A solution of (*E*)-N,N-dimethyl-3-[2-methyl-1-(4-methylbenzenesulphonyl)-1*H*-imidazol-4-yl]-2-propenamide (1.0g) in pyridine (5mℓ) and acetic anhydride (10mℓ) was stirred at room temperature for 16h. The reaction mixture was evaporated *in vacuo*, methanol (15mℓ) was added and stirring was continued for 1h. The mixture was evaporated *in vacuo* and the resulting gum partitioned between potassium carbonate solution (ca. 25% saturated) (30mℓ) and chloroform (4 x 25mℓ). The combined organic layers were dried, evaporated *in vacuo* and the residue triturated with ether (30mℓ) to give the *title compound* (340mg) as a solid, t.l.c. (ether:methanol 9:1), Rf 0.07.

Intermediate 11

***N*,*N*-Dimethyl-3-(2-methyl-1*H*-imidazol-4-yl)propanamide hydrochloride**

A solution of (*E*)-N,N-dimethyl-3-(2-methyl-1H-imidazol-4-yl)-2-propenamide (340mg) in methanol (10mℓ) and HCl-saturated ethanol (2mℓ) was added to a previously hyragen-saturated suspension of 10% palladium on charcoal (50% paste with water) (35mg) in methanol (10mℓ) and the resulting suspension stirred at room temperature under hydrogen for 4h. The mixture was filtered, concentrated *in vacuo* to *ca.* 5mℓ and ethyl acetate (ca. 40mℓ) was added, precipitating the *title compound* (275mg) as white flakes, t.l.c. (System A 89:10:1), Rf 0.14. Addition of further ethyl acetate (40mℓ) to the mother liquors precipitated further *title compound* (31mg).

Intermediate 12

Ethyl 2-chloro-3-oxohexanoate

Sulphuryl chloride (175mℓ) was added to a rapidly stirred and cooled solution of ethyl 3-oxohexanoate (300mℓ) in chloroform (150mℓ) whilst maintaining the temperature at 10-15°. The mixture was stirred overnight at room temperature, then heated under reflux for 0.5h. The cooled reaction mixture was washed

with water (0.5ℓ), 2N sodium bicarbonate (0.5ℓ) and water (0.5ℓ), dried and concentrated in vacuo. Fractionation gave the title compound (341.8g) as a pale yellow liquid b.p. 106-108°/ca. 10mmHg, t.l.c. (ether:hexane 1:1), Rf 0.60.

Intermediate 13

**Ethyl 5-propyl-1H-imidazole-4-carboxylate**

A mixture of formamide (98%, 140 mℓ), ethyl 2-chloro-3-oxohexanoate (60.0g) and water (12.5mℓ) was heated at reflux under nitrogen for 3h, cooled and partitioned between 1N hydrochloric acid (500mℓ) and ether (3x200mℓ). The acidic aqueous layer was basified with 5N sodium hydroxide (to pH8), the brown precipitate was collected by filtration and dried (vac. oven, 90°, 2h), to give the title compound (23.3g) as a brown solid, m.p. 175-179°.

Intermediate 14

**5-Propyl-1H-imidazole-4-methanol hydrochloride**

A suspension of ethyl 5-propyl-1H-imidazole-4-carboxylate (5.00g) in dry THF (250mℓ) was added to a stirred suspension of lithium aluminium hydride (1.50g) in dry THF (100mℓ) over 5 min under nitrogen. The mixture was then heated under reflux for 1h, cooled to 35° and treated sequentially with water (1.5mℓ), 15% w/v sodium hydroxide solution (4.5mℓ) and water (2.0mℓ). The mixture was filtered and evaporated in vacuo to leave an orange oil (ca. 4.3g). This oil was dissolved in absolute alcohol (5mℓ) and dry ether (200mℓ), and ethereal hydrogen chloride was added until the solution was just acidic (pH1). The mixture was evaporated in vacuo, further dry ether (150ml) was added, precipitating the title compound (3.70g) as a brown solid, m.p. 149-152°.

Intermediate 15

**5-Propyl-1H-imidazole-4-carboxaldehyde**

A mixture of 5-propyl-1H-imidazole-4-methanol (4.0g) and manganese (IV) oxide (10.0g) in 1,4-dioxan (150mℓ) was heated at reflux under nitrogen for 1h. The hot suspension was filtered (Hyflo), washed with hot 1,4-dioxan (100mℓ) and evaporated to give the title compound (2.9g) as a solid, m.p. 120-124°.

Intermediate 16

**(E)-N,N-Dimethyl-3-(5-propyl-1H-imidazol-4-yl)-2-propenamide**

A solution of dimethyl [2-(dimethylamino)-2-oxoethyl]phosphonate (1.91g) in dry THF (5mℓ) was added dropwise under nitrogen to a cold (-10°) suspension of sodium hydride (0.64g, 73% dispersion in oil) in dry THF (5mℓ). After stirring at room temperature for 1h, a solution of 5-propyl-1H-imidazole-4- carboxaldehyde (1.0g) in dry THF (10mℓ) was added at -10° and the mixture allowed to stir at room temperature for a further 18h then at reflux for 6h. The solution was poured into 8% sodium bicarbonate solution (50mℓ) and extracted with dichloromethane (3x25mℓ). The combined extracts were dried, filtered and evaporated to give an oil (1.9g). Column chromatography on silica gel (Merck 7734) eluting with ethyl acetate:methanol (17:3) gave a yellow oil (0.82g). The material was dissolved in 2N hydrochloric acid (20mℓ) and washed with ethyl acetate (2x15mℓ). The aqueous layer was basified (to pH~9) with sodium bicarbonate and extracted with dichloromethane (2x20mℓ). The combined extracts were dried, filtered and evaporated to give the title compound (0.61g) as a viscous yellow oil, t.l.c. (ethyl acetate: methanol 17:3), Rf 0.35.

Intermediate 17

**N,N-Dimethyl-3-(5-propyl-1H-imidazol-4-yl)propanamide**

A solution of (E)-N,N-dimethyl-3-(5-propyl-1H-imidazol-4-yl)-2-propenamide (500mg) in ethanol (25mℓ) was added to a suspension of pre-reduced 10% palladium oxide on carbon catalyst (150mg, 50% paste with water) in ethanol (15mℓ). The mixture was stirred in a hydrogen atmosphere for 2h, then filtered (Hyflo)

and evaporated to give the title compound (500mg) as a colourless oil, t.l.c. on $Et_3N$ impregnated $SiO_2$ - (ethyl acetate:methanol 4:1), Rf 0.33.

Intermediate 18

**(E)-3-[5-Methyl-1-(triphenylmethyl)-1H-imidazol-4-yl]-1-(1H-indol-3-yl) -2-propen-1-one**

A mixture of 5-methyl-1-(triphenylmethyl)-1H-imidazol-4-carboxaldehyde (4.90g), 3-acetylindole (2.24g) and potassium hydroxide (6.08g) in absolute ethanol (100mℓ) and water (50mℓ) was heated at 80° for 24h. The suspension was poured into 2N sodium carbonate solution (300mℓ) and extracted with dichloromethane (3x150mℓ). The combined extracts were dried, filtered and evaporated to give an orange solid (6.5g) which was triturated with ethyl acetate (50mℓ) to give the title compound (2.42g) as a yellow solid, m.p. 240-242°.

Intermediate 19

**3-[5-Methyl-1-(triphenylmethyl)-1H-imidazol-4-yl]-1-(1H-indol-3-yl)-1-propanone**

A suspension of (E)-3-[5-methyl-1-(triphenylmethyl)-H-imidazol-4-yl]-1-(1H-indol-3-yl)-2-propen-1-one (500mg) in ethanol (25 mℓ) was added to a suspension of pre-reduced 10% palladium oxide on charcoal catalyst (50mg; 50% paste with water) in ethanol (10mℓ). The mixture was hydrogenated for 120h then filtered through Hyflo and evaporated give a white solid (265mg). The Hyflo and catalyst residue was stirred with dichloromethane:ethanol (1:1, ca. 200mℓ) for 2h, filtered and evaporated to give an off-white solid (ca. 240mg). The two products were combined and purified by FCC eluting with System A (200:10:1) to give the title compound (405mg) as a white solid, m.p. 225-227°.

Intermediate 20

**1-(1-Methyl-1H-indol-3-yl)-3-[5-methyl-1-(triphenylmethyl)-1H-imidazol -4-yl]-1-propanone**

A suspension of Intermediate 19 (0.5g) in dry DMF (3mℓ) was added dropwise to a stirred suspension of sodium hydride (40mg, 73% in oil) in dry DMF (1mℓ) under nitrogen. The resulting mixture was stirred at 20° for 0.75h and treated with iodomethane (0.062mℓ) at 20° for 2.5h. The resulting yellow solution was poured into water (100mℓ) to precipitate the title compound (0.5g) as a solid, t.l.c. (System A 100:8:1), Rf 0.19.

Intermediate 21

**2-Methyl-1-(1-methyl-1H-indol-3-yl)-3-[5-methyl-1-(triphenylmethyl)-1H-imidazol-4-yl]-1-propanone**

Lithium diisopropylamide mono(tetrahydrofuran) (1.5M in cyclohexane, 11.5ml) was added dropwise to a cold (-70°) solution of 1-(1-methyl-1H-indol-3-yl)-3-[5-methyl-1-(triphenylmethyl)-1H-imidazol-4-yl]-1-propanone (8.8g) in dry THF (175mℓ) under nitrogen. The stirred solution was allowed to warm to 20° over 1h then cooled to -70°. Iodomethane (2.15mℓ) was added and the mixture was allowed to reach 20° over 1h and stirred for a further 2h. More iodomethane (1.08mℓ) was added and stirring was continued for 2h. The mixture was treated with acetic acid (10mℓ) and water (10mℓ), poured into saturated potassium carbonate solution (200mℓ) and extracted with ethyl acetate (2x100mℓ). The combined, dried organic extracts were filtered and evaporated to give a yellow foam (11.3g). FCC (column made up in EtOAc:$Et_3N$ 99:1) eluting with ethyl acetate gave the title compound (5.0g) as a white solid, m.p. 209-210°.

Intermediate 22

**3-[5-Methyl-1-(triphenylmethyl)-1H-imidazol-4-yl]-1-(2-propenyl)-1H-indol-3-yl]-1-propanone**

A suspension of Intermediate 19 (500g) in dry DMF (3mℓ) was added dropwise to a stirred suspension of sodium hydride (40mg, 73% in oil) in dry DMF (1mℓ) under nitrogen. After 15 min allyl bromide (0.105mℓ) was added and the mixture stirred for 18h. Water (50mℓ) was added and the solution extracted with dichloromethane (3x25 mℓ). The combined extracts were dried, filtered and evaporated to give a

viscous gum (685mg). Column chromatography on silica gel (Merck 7734; made up in ethyl acetate:triethylamine (99:1) with ethyl acetate as the eluent gave the title compound (245mg) as a yellow foam, t.l.c. on $Et_3N$ impregnated $SiO_2$ (ethyl acetate), Rf 0.28.

Intermediates 23 to 27 were prepared in a similar manner from Intermediate 19 and the appropriate alkylating agent. Similar column chromatography and t.l.c. conditions were used.

Intermediate 23

**1-[1-(1-Methylethyl)-1H-indol-3-yl]-3-[5-methyl-1-(triphenylmethyl)-1H-imidazol-4-yl]-1-propanone** - (740mg) as a yellow foam, t.l.c. Rf 0.35, from Intermediate 19 (1.0g) and isopropyl iodide (0.24ml) with a reaction time of 16h.

Intermediate 24

**1-(1-Cyclopentyl-1H-indol-3-yl)-3-[5-methyl-1-(triphenylmethyl)-1H-imidazol-4-yl]-1-propanone** - (370mg) as a yellow foam, t.l.c. Rf 0.40, from Intermediate 19 (500mg) and cyclopentylbromide (0.13ml) with a reaction time of 29h.

Intermediate 25

**1-(1-Cyclopentylmethyl-1H-indol-3-yl)-3-[5-methyl-1-(triphenylmethyl)-1H-imidazol-4-yl]-1-propanone** (365mg) as a yellow foam, t.l.c. Rf 0.40, from Intermediate 19 (500mg) and cyclopentanemethanol (4-methylbenzenesulphonate) (305mg) with a reaction time of 22h.

Intermediate 26

**3-[5-Methyl-1-(triphenylmethyl)-1H-imidazol-4-yl]-1-(1-phenylmethyl-1H- indol-3-yl)-1-propanone** - (175mg) as a white solid, m.p. 186-188°, from Intermediate 19 (500mg) and benzyl bromide (0.14ml) with a reaction time of 20h.

Intermediate 27

**3-[5-Methyl-1-(triphenylmethyl)-1H-imidazol-4-yl]-1-[1-(2-propynyl)-1H- indol-3-yl)-1-propanone** - (37mg) as a yellow foam, t.l.c. Rf 0.37, from Intermediate 19 (250mg), potassium carbonate (138mg) (instead of sodium hydride) and propargyl bromide (0.045ml) in acetone with a reaction time of 96h.

Intermediate 28

**(E)-1-(1-Methyl-1H-indol-3-yl)-3-[5-methyl-1-(triphenylmethyl)-1H-imidazol-4-yl]-2-propen-1-one**

A mixture of 5-methyl-1-(triphenylmethyl)-1H-imidazole-4-carboxaldehyde (2.5g), 1-(1-methyl-1H-indol-3-yl)-1-ethanone (1.0g) and potassium hydroxide (3.1g) in absolute ethanol (50mℓ) and water (25mℓ) was stirred at room temperature for 72h, then at 50° for 18h. The mixture was partitioned between 2N sodium carbonate (200mℓ) and ethyl acetate (2 x 200mℓ) and the combined organic layers were washed with brine (150mℓ), dried and evaporated in vacuo to leave a foam (3.9g) which was purified by FCC eluting with ethyl acetate:hexane (1:1) to give the title compound (1.76g) as a solid, m.p. 216-219°.

Intermediate 29

**1-Methyl-[3-[3-(5-methyl-1H-imidazol-4-yl)propyl]]-1H-indole maleate**

Diisobutyl aluminium hydride (1.0M in hexane, 1.1m) was added to a stirred, cold (-60°) solution of 3-(5-methyl-1H-imidazol-4-yl)-1-(1-methyl-1H-indol-3-yl)-1-propanone (0.83g) in dry THF (40mℓ) under nitrogen. After 2h, the mixture had warmed to 0°, and was quenched with brine (50mℓ). Dichloromethane (300mℓ) was added and the mixture was filtered. The organic layer of the filtrate was dried and evaporated in vacuo to leave an orange oil (ca. 1g). This oil was purified by FCC eluting with System A (92.5:7.5:0.75) to give the free base of the title compound (0.3g) as an orange gum. This gum was dissolved in ethanol (5mℓ), and treated with a solution of maleic acid (160mg) in hot ethanol (1mℓ). The mixture was evaporated and dissolved in hot ethyl acetate (100mℓ). Further purification by FCC eluting with System A (94.5:5:0.5) gave the free base of the title compound (60mg) as a yellow oil. This oil was dissolved in ethyl acetate (15mℓ), and a solution of maleic acid (32mg) in ethyl acetate (1mℓ) was added. Dilution with dry ether (15mℓ) and cooling to 4° overnight gave the title compound (75mg) as white crystals, m.p. 84-85°.

Intermediate 30

**4-(Chloromethyl)-5-methyl-1-(triphenylmethyl)-1H-imidazole**

A solution of thionyl chloride (1.3mℓ) in dry dichloromethane (10mℓ) was added over 5min to a stirred suspension of 5-methyl-1-(triphenylmethyl)-1H-imidazole-4-methanol (5.0g) in a mixture of dichloromethane (100mℓ) and dry DMF (2mℓ) at 0°. The mixture was stirred at 0° for 0.5h and washed consecutively with 8% sodium bicarbonate (2 x 50mℓ), water (50mℓ), dried and evaporated in vacuo below 40° to give an oil (5g). This was dissolved in ether (100mℓ) and the resulting solution filtered through a pad of silica which was further eluted with ether (2 x 100mℓ). The combined filtrates were evaporated below 40° to give a white foam which was triturated with cold hexane and filtered to give the title compound (4.2g) as a white crystalline solid, m.p. 133-135°.

Example 1

**3-(1H-Imidazol-4-yl)-1-(1-methyl-1H-indol-3-yl)-1-propanone maleate**

Phosphorus oxychloride (10mℓ) was added over 5min to N,N-dimethyl-3-(1H-imidazol-4-yl)-1-propanamide hydrochloride (750mg) under nitrogen at 0°. The reaction mixture was stirred and heated at 40° for 1h, and the excess phosphorus oxychloride was removed by evaporation in vacuo. 1,2-Dichloroethane (30mℓ) was added followed by 1-methylindole (0.47mℓ) and the mixture was heated under nitrogen at reflux for 1h. The mixture was cooled and phosphorus oxychloride (0.34mℓ) was added. It was then stirred for 0.5h and heated at reflux for 2h. The cooled reaction mixture was poured onto ice (ca. 150mℓ) and stirred overnight. The aqueous solution was washed with dichloromethane (2x75mℓ; discarded), basified with 2N sodium carbonate and extracted with dichloromethane (3x75mℓ). The combined, dried organic extracts were evaporated in vacuo to give a foam (0.64g). This was dissolved in absolute ethanol (25mℓ), and maleic acid (323mg) in methanol (3mℓ) was added with stirring. Dry ether (ca. 25 mℓ) was added, precipitating the crystalline title compound (842mg) after drying (pistol, 76°, 18h), m.p. 158-161°.
Analysis Found : C,61.5;H,5.1;N,11.3;
$C_{15}H_{15}N_3O.C_4H_4O_4$ requires C,61.8;H,5.2;N,11.4%.

Example 2

**3-(1H-Imidazol-4-yl)-1-(1,2-dimethyl-1H-indol-3-yl)-1-propanone maleate**

Phosphorus oxychloride (0.10mℓ) was added to a mixture of N,N-dimethyl-3-(1H-imidazol-4-yl)-propanamide hydrochloride (220mg) and 1,2-dimethylindole (142mg) and the mixture was heated at 85° for 20h. Cold water (40mℓ) was added and the cooled mixture was washed with dichloromethane (2x20mℓ; discarded), basified with 2N sodium carbonate and extracted with dichloromethane (4x20mℓ). The combined, dried organic extracts were evaporated to yield a foam (0.22g). This was purified by FCC eluting with System A (89:10:1) to give the free base of the title compound (160mg) as a yellow oil. This was dissolved in ethanol (20mℓ), and maleic acid (70mg) in ethanol (2mℓ) was added. The solution was evaporated and the solid residue crystallised from ethanol (8mℓ) to give the title compound (173mg) as white crystals, m.p. 143-144°.
Analysis Found : C,62.2; H,5.8; N,10.7;
$C_{16}H_{17}N_3O.C_4H_4O_4.0.1C_2H_5OH$ requires C,62.5; H,5.6; N,10.8%.

Example 3

**3-(5-Methyl-1H-imidazol-4-yl)-1-(1-methyl-1H-indol-3-yl)-1-propanone maleate**

A mixture of N,N-dimethyl-3-(5-methyl-1H-imidazol-4-yl)propanamide dihydrochloride (1.8g), 1-methylindole (1.12g) and phosphorus oxychloride (1.01mℓ) was stirred at 85° under nitrogen for 1.75h. The mixture was cooled and water (ca. 60mℓ) was added. The aqueous solution was washed with dichloromethane (2x30mℓ; discarded), basified with 2N sodium carbonate (to pH 9) and extracted with dichloromethane (3x40mℓ). The combined organic extracts were evaporated in vacuo to give a gum (1.33g). Maleate formation as in Example 22 gave the title compound (1.3g) as a solid, m.p. 150-153°. This material was identical to the product from Example 22 by t.l.c.

Example 4

**3-(5-Methyl-1H-imidazol-4-yl)-1-(1,2-dimethyl-1H-indol-3-yl)-1-propanone maleate**

A mixture of N,N-dimethyl-3-(5-methyl-1-H-imidazol-4-yl)propanamide dihydrochloride (393mg), 1,2-dimethylindole (270mg) and phosphorus oxychloride (0.19mℓ) was heated at 85° in a closed vessel for 3h. The mixture was cooled and cold water (50mℓ) was added. The aqueous solution was washed with dichloromethane (2x50mℓ; discarded), basified with 2N sodium carbonate and extracted with ethyl acetate (5x150mℓ). The combined, dried organic extracts were evaporated to yield the free base of the title compound as an oily solid (470mg). This was purified by FCC eluting with System A (89:10:1) to give the free base of the title compound (438mg) as an oily solid. This was dissolved in hot ethanol (50mℓ), and maleic acid (198mg) in warm ethanol (5mℓ) was added. The solution was evaporated and the residual solid was crystallised from ethanol (20mℓ) to give the title compound (448mg) as white crystals, m.p. 170-171°.
Analysis Found : C,63.4; H,5.8; N,10.6;
$C_{17}H_{19}N_3O.C_4H_4O_4$ requires C,63.5; H,5.8; N,10.6%.

Example 5

**3-(5-Methyl-1H-imidazol-4-yl)-1-(1-methyl-2-phenyl-1H-indol-3-yl)-1-propanone maleate**

A solution of N,N-dimethyl-3-(5-methyl-1H-imidazol-4-yl)propanamide (250mg) in ethanol (10mℓ) was acidified (to pH 1) by the addition of ethanolic hydrogen chloride. The yellow solution was evaporated and the residual oil triturated with ether (10mℓ) and evaporated to give N,N-dimethyl-3-(5-methyl-1H-imidazol-4-yl)propanamide dihydrochloride as white crystals. 1-Methyl-2-phenylindole (343mg) and phosphorus oxychloride (0.19mℓ) were added and the mixture heated at 85° for 1.5 h. The mixture was cooled, water (60mℓ) was added and the aqueous solution was washed with dichloromethane (2x50mℓ; discarded), basified with 2N sodium carbonate (to pH 10) and extracted with ethyl acetate (2x85mℓ). The combined, dried organic extracts were evaporated to give a yellow oil. This was purified by SPCC eluting with System A (956:40:4) to give the free base of the title compound (193mg) as a yellow oil (183mg). This yellow oil was dissolved in hot ethanol (20mℓ), and maleic acid (67mg) in ethanol (3mℓ) was added. The solution was evaporated and the residual solid was crystallised from ethanol to give the title compound (153mg) as a white powder, m.p. 166-167°.
Analysis Found : C,67.9; H,5.5; N,9.1;
$C_{22}H_{21}N_3O.C_4H_4O_4$ requires C,68.0; H,5.5; N,9.1%.

Example 6

**3-(2-Methyl-1H-imidazol-4-yl)-1-(1-methyl-1H-indol-3-yl)-1-propanone maleate**

Phosphorus oxychloride (10mℓ) was added dropwise over 15 min to N,N-dimethyl-3-(2-methyl-1H-imidazol-4-yl)propanamide hydrochloride (250mg) at 0° under nitrogen and the mixture stirred at 40° for 1.25h. The resulting suspension was evaporated in vacuo while protecting from atmospheric water, and dry 1,2-dichloroethane (20mℓ) followed by further phosphorus oxychloride (0.10mℓ) were added to the remaining solid. The mixture was heated under nitrogen to 80° and 1-methylindole (0.15mℓ) added. The reaction was heated under reflux for 3h, cooled, poured onto ice (ca. 150mℓ) and left overnight. 2N Hydrochloric acid (20mℓ) and dichloromethane (75mℓ) were added to the resulting mixture and the layers separated. The aqueous layer was basified with saturated potassium carbonate and extracted with dichloromethane (3x100mℓ). These combined organic layers were dried and evaporated in vacuo to give a foam (0.2g). This foam was dissolved in ethanol (5mℓ), and a solution of maleic acid (108mg) in ethanol (1.5mℓ) was added with stirring. Addition of ethyl acetate (5mℓ) precipitated the crystalline title compound (220mg), m.p. 166-168°.
Analysis Found : C,62.5; H,5.4; N,10.8;
$C_{16}H_{17}N_3O.C_4H_4O_4$ requires C,62.65; H,5.5; N,11.0%.

Example 7

**1-(1-Methyl-1H-indol-3-yl)-3-(5-propyl-1H-imidazol-4-yl)-1-propanone maleate**

A solution of N,N-dimethyl-3-(5-propyl-1H-imidazol-4-yl)propanamide (500mg) in dichloromethane (5mℓ) was treated with an excess of ethereal hydrogen chloride. The solvent was removed in vacuo and the residue triturated with dry ether (3 x 10mℓ) to leave a viscous gum. 1-Methylindole (0.31mℓ) and phosphorus oxychloride (0.26mℓ) were added and the mixture heated at 80° for 1.5h. Water (50mℓ) was added and the suspension was washed with dichloromethane (2 x 50mℓ; discarded), basified with 2N sodium carbonate (to pH 9) and extracted with dichloromethane (3 x 50mℓ). The combined, dried organic extracts were evaporated to give a yellow foam (ca. 600mg). FCC eluting with System A (200:10:1) gave a yellow foam (330mg). This was dissolved in ethanol (ca. 5mℓ), and maleic acid (132mg) in ethanol (ca. 1.5mℓ) was added. The solvent was removed in vacuo and the residue triturated with dry ether (3 x 10mℓ) to give the title compound (420mg) as a solid, m.p. 144-146°.
Analysis Found : C,64.1; H,6.2; N,10.0
$C_{18}H_{21}N_3O.C_4H_4O_4$ requires C,64.2;H,6.1; N,10.2%.

Example 8

**2,2-Dimethyl-3-(5-methyl-1H-imidazol-4-yl)-1-(1-methyl-1H-indol-3-yl) 1-propanone maleate**

Lithium diisopropylamide mono(tetrahydrofuran)(1.5M in cyclohexane, 2.55mℓ) was added dropwise to a cold (-70°) solution of 2-methyl-1-(1-methyl-1H-indol-3-yl)-3-[5-methyl-1-triphenylmethyl)-1H-imidazol-4-yl]-1-propanone (2.0g) in dry THF (40mℓ) under nitrogen. Hexamethylphosphoramide (0.665mℓ) was added and the stirred solution was allowed to warm to 20° over 1.5h. The solution was cooled (-70°) and iodomethane (0.24mℓ) was added. The mixture was allowed to warm to 0° over 1h and was stirred at this temperature for 2h. Acetic acid (4mℓ) and water (4mℓ) were added and the mixture was poured into saturated potassium carbonate solution (80mℓ) and extracted with ethyl acetate (2x40mℓ). The combined, dried organic extracts were filtered and evaporated to give a yellow oil (ca. 3.5 g). The crude material was dissolved in acetic acid (25mℓ), water (25mℓ) and THF (25mℓ), and heated at reflux for 2h. The THF was removed in vacuo, the residual suspension was dissolved in 1N hydrochloric acid (200mℓ) and washed with ethyl acetate (2x100mℓ). The combined organic layers were extracted with 1N hydrochloric acid (100mℓ) and then discarded. The combined aqueous layers were basified (to pH 9) with potassium carbonate and extracted with dichloromethane (3x100mℓ). The combined extracts were dried, filtered and evaporated to give a dark green oil (ca. 2g). SPCC eluting with System A (200:10:1) gave a yellow foam (670mg). This material was dissolved in dichloromethane (ca. 5mℓ) and a solution of maleic acid (267mg) in ethanol (ca. 2mℓ) was added. The solvent was removed in vacuo and the residue triturated with dry ether (3x20mℓ) to give the title compound (805mg) as a white solid, m.p. 119-120°.
Analysis Found : C,64.3; H,6.1; N,10.0;
$C_{18}H_{21}N_3O.C_4H_4O_4$ requires C,64.2; H,6.1; N,10.2%.

Example 9

**2-Methyl-3-(5-methyl-1H-imidazol-4-yl)-1-(1-methyl-1H-indol-3-yl)-1-propanone maleate**

A solution of lithium diisopropylamide was made by the addition of n-butyllithium (1.56M in hexane, 0.64mℓ) to a cold (0°) solution of diisopropylamine (0.07mℓ) in dry THF (9.3mℓ). A portion of this solution (9.3mℓ) was added to a stirred cold (-70°). solution of 1-(1-methyl-1H-indol-3-yl)-3-[5-methyl-1-(triphenylmethyl)-1H-imidazol-4-yl]-1-propanone (0.5g) in dry THF (10mℓ) under nitrogen. The resulting mixture was stirred at -70° for 1h and allowed to warm to 20° (ca .1h). The mixture was then cooled to -70° and treated with iodomethane (0.1mℓ) stirred for 1h and allowed to warm to 20°. A further portion of iodomethane (0.1mℓ) was added and the mixture was stirred at 20° for 2h and then quenched with acetic acid (10mℓ) and water (10mℓ). The acidic mixture was partitioned between saturated potassium carbonate solution (60mℓ) and ethyl acetate (2x60mℓ). The combined, dried organic extracts were evaporated to give a brown gum (0.5g), which was purified by SPCC eluting with ethyl acetate: methanol:triethylamine (80:20:1) to give a white foam (0.33g). This was dissolved in a mixture of acetic acid (9mℓ) and water (9mℓ) and heated on a steam bath for 2.5h. The resulting mixture was cooled to 20° and partitioned between ethyl acetate (3x60mℓ) and saturated potassium carbonate solution (60mℓ). The combined, dried organic extracts were evaporated to leave a brown gum (0.35g) which was purified by FCC eluting with System A (100:8:1) to give a white foam. This foam was dissolved in ethyl acetate (12mℓ) and treated with a solution of maleic acid (27mg) in ethyl acetate (3mℓ) to precipitate the title compound (0.09g) as a white solid, m.p. 161-163°.
Analysis Found : C,63.3; H,5.9; N,10.3;

$C_{17}H_{19}N_3O.C_4H_4O_4$ requires C,63.5; H,5.8; N,10.6%.

Example 10

**3-(1-Methyl-1H-imidazol-4-yl)-1-(1-methyl-1H-indol-3-yl)-1-propanone maleate**

3-(1H-Imidazol-4-yl)-1-(1-methyl-1H-indol-3-yl)-1-propanone maleate (440mg) was partitioned between 8% sodium hydrogen carbonate (75ℓ) and chloroform (3x50mℓ) and the combined, dried organic layers were evaporated in vacuo to leave a foam (300mg). This foam was dissolved in dry DMF (15mℓ) at 0° under nitrogen and sodium hydride (42mg, 78% in oil) was added with stirring. After 15 min, methyl iodide (0.075mℓ) was added and stirring was continued for 1h at 0°. Saturated sodium hydrogen carbonate (10mℓ) was added and the resulting suspension partitioned between water (75mℓ) and chloroform (3x75mℓ). The combined, dried organic layers were evaporated in vacuo and the residual gum was purified by FCC eluting with System A (94.5:5:0.5) followed by high pressure liquid chromatography (h.p.l.c.) (5µm Hypersil column 25cm x 4.6mm_ eluting with n-hexane:chloroform:ethanol (100:100:10) + 0.1% $NH_4OH$ to give a gum (118mg) as the first eluted u.v. active component. This gum was dissolved in absolute ethanol (1.5mℓ), and a solution of maleic acid (54mg) in absolute ethanol (0.5mℓ) was added. Dilution with ethyl acetate (ca. 4mℓ) precipitated the title compound (113mg) as a crystalline solid, m.p. 136-137°.
Analysis Found : C,62.4; H,5.5; N,10.8;
$C_{16}H_{17}N_3O.C_4H_4O_4$ requires C,62.6; H,5.5; N,11.0%.

Example 11

**3-(1-Methyl-1H-imidazol-5-yl)-1-(1-methyl-1H-indol-3-yl)-1-propanone maleate**

The h.p.l.c. of Example 10 gave a solid (83mg) as the second eluted u.v. active compound. This solid was dissolved in absolute ethanol (1.5mℓ), filtered, and a solution of maleic acid (38mg) in absolute ethanol (0.5mℓ) was added. Dilution with ethyl acetate (ca. 4mℓ) precipitated the title compound (80mg) as a crystalline solid, m.p. 157-158°.
Analysis Found : C,62.2; H,5.4; N,10.7;
$C_{16}H_{17}N_3O.C_4H_4O_4$ requires C,62.6; H,5.5; N,11.0%.

Examples 12a and 12b

**1-(1-Methyl-1H-indol-3-yl)-3-[5-methyl-1-(2-propenyl)-1H-imidazol-4-yl]-1-propanone maleate (12a) and 1-(1-Methyl-1H-indol-3-yl)-3-4-methyl-1-(2-propenyl)-1H-imidazol-5-yl]-1-propanone maleate (12b)**

A solution of 3-(5-methyl-1H-imidazol-4-yl)-1-(1-methyl-1H-indol -3-yl)-1-propanone (500mg) in dry DMF (3mℓ) was added dropwise to a stirred suspension of sodium hydride (74mg, 73% in oil) in dry DMF (1.5mℓ). After 20 min allyl bromide (0.16mℓ) was added and the mixture was stirred under nitrogen for 1.5h. Water (50mℓ) was added and the suspension extracted with dichloromethane (3x25mℓ). The combined dried organic extracts were filtered and evaporated to give a yellow oil (ca. 800mg). FCC eluting with System A (400:10:1) gave a mixture of the free bases of the title compounds (12a) and (12b) (310mg) as a brown oil. Purification by h.p.l.c. (Zorbax 7-8 µm silica, 250 x 21.2mm column) eluting with chloroform:n-hexane:methanol (200:40:15) + 0.4% water gave the separated free bases of the title compounds (12a) (166mg) and (12b) (62mg) respectively. Maleate formation from each of these compounds as in Example 8 gave the title compound (12a) (162mg) as a solid, m.p. 105-106°.
Analysis Found: C,64.95; H,5.95; N,9.81;
$C_{19}H_{21}N_3O.C_4H_4O_4$ requires C,65.23; H,5.95; N,9.92%;
and the title compound (12b) (67mg) as a solid, m.p. 108-109°.
Water Analysis Found: 0.76% w/w ≡ 0.17 mol $H_2O$
Analysis Found : C,64.7; H,6.0; N,9.6;
$C_{19}H_{21}N_3O.C_4H_4O_4.O_4.0.13H_2O$ requires C,64.8; H,6.0; N,9.85%.

Examples 13a and 13b

**1-(1-Methyl-1H--indol-3-yl)-3-[5-methyl-1-(phenylmethyl)-1H-imidazol-4-yl]-1-propanone maleate**

**(13a) and 1-(Methyl-1H-indol-3-yl)-3-[4-methyl-1-(phenylmethyl)-1H-imidazol-5-yl]-1-propanone maleate (13b)**

Examples (13a) and (13b) were prepared from 3-(5-methyl-1H-imidazol-4-yl)-1-(1-methyl-1H-indol-3-yl)-1-propanone (500mg) and benzyl bromide (0.22mℓ) using the method of Examples (12a) and (12b) Similar FCC and t.l.c. conditions were used, to give a mixture of the free bases of the title compounds (13a and 13b) (470mg) as a yellow foam. Purification by h.p.l.c. (using the conditions of Examples (12a) and (12b)) gave the separated free bases of the title compounds (13a) (275mg) and (13b) (113mg) respectively. Maleate formation from each of these compounds as in Example 8 gave the title compound (13a) (317mg) as a solid, m.p. 128-129°.
Analysis Found : C68.37; H,5.79; N,8.70;
$C_{23}H_{23}N_3O.C_4H_4O_4$ requires C,68.48; H,5.75; N,8.87%;
and the title compound (13b) (121mg) as a solid, m.p. 138-140°.
Water Analysis Found : 0.92% w/w ≡ 0.24 mol $H_2O$
Analysis Found : C,67.9; H,5.9; N,8.7;
$C_{23}H_{23}N_3O.C_4H_4O_4.0.24H_2O$ requires C67.9; H,5.8; N,8.8%.

Example 14

**3-(5-Methyl-1H-imidazol-4-yl)-1-(1H-indol-3-yl)-1-propanone maleate**

A solution of 3-[5-methyl-1-(triphenylmethyl)-1H-imidazol-4-yl]-1-(1H-indol-3-yl)-1-propanone (370mg) in acetic acid (10mℓ), water (10mℓ) and THF (10mℓ) was heated at reflux for 1h. The solution was poured into 1N hydrochloric acid (50mℓ) and washed with ethyl acetate (50mℓ; discarded). The aqueous layer was basified with potassium carbonate (to pH ~9) and extracted with dichloromethane (3x50mℓ). The combined, dried organic extracts were filtered and evaporated to give a yellow solid (130mg). This was dissolved in ethanol (3mℓ), and maleic acid (62mg) in ethanol (0.5mℓ) was added. The solvent was removed in vacuo and the residue triturated with dry ether (4x10mℓ) to give the title compound (173mg) as a white solid, m.p. 125°.
Analysis Found : C,61.3; H,5.4; N,10.8;
$C_{15}H_{15}N_3O.C_4H_4O_4.0.2C_2H_5OH$ requires C,61.6; H,5.3; N,11.1%.

Examples 15 to 21 were prepared in a similar manner from the appropriate protected intermediates.

Example 15

**3-(5-Methyl-1H-imidazol-4-yl)-1-[1-(2-propenyl)-1H-indol-3-yl]-1-propanone maleate**

The deprotection of 3-[5-methyl-1-(triphenylmethyl)-1H-imidazol-4-yl]-1-[1-(2-propenyl)-1H-indol-3-yl]-1-propanone (215mg) gave the title compound (80mg) as a pale brown solid, m.p. 142-144°.
Analysis Found : C,64.7; H,5.9; N,9.9;
$C_{18}H_{19}N_3O.C_4H_4O_4$ requires C,64.5; H,5.7; N,10.3%.

Example 16

**1-[1-(1-Methylethyl)-1H-indol-3-yl]-3-(5-methyl-1H-imidazol-4-yl)-1-propanone maleate**

The deprotection of 1-[1-(1-methylethyl)-1H-indol-3-yl]-3-[5-methyl-1-(triphenylmethyl)-1H-imidazol-4-yl]-1-propanone (670mg) gave the title compound (295mg) as a pale brown solid, m.p. 148-150°.
Analysis Found : C,64.2; H,6.1; N,10.1;
$C_{18}H_{21}N_3O.C_4H_4O_4$ requires C,64.2; H,6.1; H,10.2%.

Example 17

**1-(1-Cyclopentyl-1H-indol-3-yl)-3-(5-methyl-1H-imidazol-4-yl)-1-propanone maleate**

The deprotection of 1-(1-cyclopentyl-1H -indol-3-yl)-3-[5-methyl-1-(triphenylmethyl)-1H-imidazol-4-yl]-1-propanone (340mg) gave the title compound (120mg) as a white solid, m.p. 86-89°, t.l.c. (System A 200:10:1), Rf 0.33.

Example 18

**1-(1-Cyclopentylmethyl-1H-indol-3-yl)-3-(5-methyl-1H-imidazol-4-yl)-1-propanone maleate**

The deprotection of 1-(1-cyclopentylmethyl-1H-indol-3-yl)-3-[5-methyl-1-(triphenylmethyl)-1H-imidazol-4-yl]-1-propanone (300mg) gave the title compound (87mg) as a solid, m.p. 158-160°.
Water Analysis Found: 0.285% w/w ≡ 0.072mol $H_2O$
Analysis Found: C,65.9; H,6.4; N,9.4;
$C_{21}H_{25}N_3O.C_4H_4O_4.0.072H_2O$ requires C,66.3; H,6.5; N,9.3%.

Example 19

**3-(5-Methyl-1H-imidazol-4-yl)-1-[1-(phenylmethyl)-1H-indol-3-yl]-1-propanone maleate**

The deprotection of 3-[5-methyl-1-(triphenylmethyl)-1H-imidazol-4-yl]-1-[1-(phenylmethyl)-1H-indol-3-yl]-1-propanone (160mg) gave, after purification of the combined products of the dichloromethane extracts (37mg) and the ethyl acetate wash (150mg) by column chromatography on silica gel (Merck 7734; made up in EtOAc:MeOH:$Et_3N$ 90:9:1) eluting with ethyl acetate:methanol (9:1), a colourless oil (83mg). Maleate formation gave the title compound (100mg) as a white solid, m.p. 145-147°, t.l.c. on $Et_3N$ impregnated $SiO_2$ (EtOAc:MeOH 9:1), Rf 0.39.

Example 20

**3-(5-Methyl-1H-imidazol-4-yl)-1-[1-(2-propynyl)-1H-indol-3-yl]-1-propanone maleate**

The deprotection of 3-[5-methyl-1-(triphenylmethyl)-1H-imidazol-4-yl]-1-[1-(2-propynyl)-1H-indol-3-yl]-1-propanone (30mg) using the method of Example 14, but adding the reaction mixture to saturated potassium carbonate solution gave, after purification of the product of the dichloromethane extracts (30mg) by FCC eluting with System A (150:10:1), a brown oil (10mg). Maleate formation gave the title compound (6mg) as a solid, m.p. 155-158°, t.l.c. (System A 150:10:1), Rf. 0.22.

Example 21

**(E)-3-(5-Methyl-1H-imidazol-4-yl)-1-(1-methyl-1H-indol-3-yl)-2-propen-1-one maleate**

The deprotection of (E)-1-(1-methyl-1H-indol-3-yl)-3-[5-methyl-1-(triphenylmethyl)-1H-imidazol-4-yl]-2-propen-1-one (300mg) was effected using the method of Example 14, with the exception that the reaction mixture was poured into 0.1N hydrochloric acid and extracted with ethyl acetate. Maleate formation followed by the addition of ethyl acetate, precipitated the title compound (195mg) as yellow crystals, m.p. 190-192°, t.l.c. (ethyl acetate:hexane 1:1), Rf 0.6.

Example 22

**3-(5-Methyl-1H-imidazol-4-yl)-1-(1-methyl-1H-indol-3-yl)-1-propanone maleate**

A solution of (E)-1-(1-methyl-1H-indol-3-yl)-3-[5-methyl-1-(triphenylmethyl)-1H-imidazol-4-yl]-2-propen-1-one (1.4g) in ethyl acetate (150ml) and ethanol (50ml) was added to a previously hydrogenated suspension of 10% palladium on carbon (140mg of a 50% paste with water) in ethanol (50ml) and was stirred under hydrogen for 64h. The reaction mixture was filtered through Hyflo, evaporated in vacuo and the residual gum was triturated with hexane (100mℓ). The residue was treated with ethyl acetate (5mℓ) followed by hexane (100mℓ), and the resulting precipitate was filtered off. This solid was dissolved in THF (7mℓ), acetic acid (7mℓ) and water (7mℓ), heated at reflux for 1h and the cooled solution was partitioned between 1N hydrochloric acid (2 x 100mℓ) and ethyl acetate (100mℓ). The aqueous layers were basified with potassium carbonate (to pH9), and extracted with dichloromethane (3 x 100mℓ). The combined, dried organic extracts were evaporated in vacuo to give a solid (0.75g). This solid was dissolved in hot ethanol (25mℓ) and chloroform (10mℓ), and maleic acid (340mg) in ethanol (10mℓ) was added. The solution was concentrated in vacuo to ca. 15mℓ and diluted with ether to give the crystalline title compound (0.85g), m.p. 155-156°, t.l.c. (System A 89:10:1), Rf. 0.22.

Water Analysis Found : 0.62% w/w ≡ 0.13 mol $H_2O$
Analysis Found : C,61.9; H,5.3; N,10.9;
$C_{16}H_{17}N_3O.C_4H_4O_4.0.13H_2O$ requires C,62.3; H,5.5; N,10.9%.
Further concentration in vacuo of the mother liquors gave further title compound (40mg) as a solid, m.p. 153-156°.

Example 23

**3-(5-Methyl-1H-imidazol-4-yl)-1-(1-methyl-1H-indol-3-yl)-1-propanone**

1-Methyl-[3-[3-(5-methyl-1H-imidazol-4-yl)propyl]]-1H-indole maleate (15mg) was partitioned between 2N sodium carbonate (10ml) and dichloromethane (3 x 10ml). The combined, dried organic layers were evaporated in vacuo to leave a clear gum. To a stirred solution of this gum in 10% aqueous THF (2ml) was added a solution of 2,3-dichloro-5,6-dicyano-1,4-benzoquinone in dry THF (0.8mℓ), dropwise at -10 to -15° under nitrogen. After 1.5h the reaction mixture was evaporated in vacuo and purified by FCC eluting with System A (94.5:5:0.5) to give the title compound (6.7mg) as a gum, t.l.c. (System A 89:10:1), Rf. 0.27.
N.m.r. δ ($CDCl_3$) 2.20 (3H,s,C=C-$CH_3$), 2.98 (2H,m,$CH_2$-Im), 3.20 (2H, m, $CH_2$-CO-), 3.83 (3H,s,N-$CH_3$), 7.30-7.38 and 8.38 (3H, m and 1H, m respectively, benzene ring protons), 7.42 (1H, s, CH=N of imidazole), 7.73 (1H, s, N-CH of indole).

Example 24

**3-(5-Methyl-1H-imidazol-4-yl)-1-(1-methyl-1H-indol-3-yl)-1-propanone maleate**

n-Butyllithium (1.58M in hexane, 4.1mℓ) was added at -78° under nitrogen to a stirred solution of diisopropylamine (0.9mℓ) in dry THF (25mℓ) and the solution was stirred at 0° for 0.5 h. The solution was cooled to -78° and added via a cannula to a stirred solution of 3-acetyl-1-methylindole (928mg) in dry THF (5mℓ) at -78° under nitrogen. After 0.5h at -78° the solution was stirred at 0° for 0.5h, cooled to -78° and a solution of 4-(chloromethyl)-5-methyl-1-(triphenylmethyl)-1H-imidazole (2.00g) in dry THF (20mℓ) was added dropwise with stirring under nitrogen. The solution was stirred at -78° for 1h and at 0° for 2h, poured into cold 8% aqueous sodium bicarbonate (100mℓ) and extracted with ethyl acetate (2x100mℓ). The combined, dried organic extracts were evaporated to give an orange oil (ca. 3g) which was treated with a mixture of glacial acetic acid (40mℓ), water (40mℓ) and THF and heated at reflux for 1h. The solution was evaporated and treated with 1N hydrochloric acid (70mℓ). It was then washed with ethyl acetate (70mℓ; discarded), basified with 2N sodium carbonate (to pH 11) and extracted with dichloromethane (4x130mℓ). The combined, dried organic extracts were evaporated to give a brown foam (350mg) which was purified by SPCC eluting with System A (923:70:7) to give the free base of the title compound as a pale yellow oil (81mg). Maleate formation as in Example 22, but using ethanol alone as the solvent gave the title compound (50mg) as a solid, m.p. 148-150°. This material was identical to the product from Example 22 by t.l.c.

The following examples illustrate pharmaceutical formulations according to the invention. The term "active ingredient" is used herein to represent a compound of formula (I).

**TABLETS FOR ORAL ADMINISTRATION**

Tablets may be prepared by the normal methods such as direct compression or wet granulation.

The tablets may be film coated with suitable film forming materials, such as hydroxypropyl methylcellulose, using standard techniques. Alternatively the tablets may be sugar coated.

Direct Compression

Direct Compression

| Tablet | mg/tablet |
|---|---|
| Active Ingredient | 0.50 |
| Calcium Hydrogen Phosphate BP* | 87.25 |
| Croscarmellose Sodium NF | 1.8 |
| Magnesium Stearate BP | 0.45 |
| Compression weight | 90.0 |

* of a grade suitable for direct compression.

The active ingredient is passed through a 60 mesh sieve, blended with the calcium hydrogen phosphate, croscarmellose sodium and magnesium stearate. The resultant mix is compressed into tablets using a Manesty F3 tablet machine fitted with 5.5mm, flat bevelled edge punches.

Tablets of other strengths may be prepared by altering the ratio of active ingredient to excipients or the compression weight and using punches to suit.

CAPSULES

| CAPSULES | mg/~~tablet~~ capsule |
|---|---|
| Active Ingredient | 0.5 |
| * Starch 1500 | 98.5 |
| Magnesium Stearate BP | 1.0 |
| Fill Weight | 100.0 |

* a form of directly compressible starch.

The active ingredient is sieved and blended with the excipients. The mix is filled into size No. 2 hard gelatin capsules using suitable machinery. Other doses may be prepared by altering the fill weight and if necessary changing the capsule size to suit.

SYRUP

The may be either a sucrose or sucrose free presentation.

| Sucrose-Free | mg/5ml dose |
|---|---|
| Active Ingredient | 0.5 |
| Hydroxypropylmethylcellulose USP (viscosity type 4000) | 22.5 |
| Buffer ) | |
| Flavour ) | |
| Colour ) | as required |
| Preservative ) | |
| Sweetener ) | |
| Purified Water BP to | 5.0ml |

The hydroxypropylmethylcellulose is dispersed in hot water, cooled and then mixed with an aqueous solution containing the active ingredient and the other components of the formulation. The resultant solution is adjusted to volume and mixed. The syrup is clarified by filtration.

Injection for Intravenous Administration

| | mg/ml | |
|---|---|---|
| Active ingredient | 0.05 | 0.5 |
| Sodium Chloride BP | as required | as required |
| Water for Injection BP to | 1.0ml | 1.0ml |

Sodium chloride may be added to adjust the tonicity of the solution and the pH may be adjusted, using acid or alkali, to that of optimum stability and/or facilitate solution of the active ingredient. Alternatively suitable buffer salts may be used.

The solution is prepared, clarified and filled into appropriate size ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen or other suitable gas.

## Claims

1. Compounds of the general formula (I):

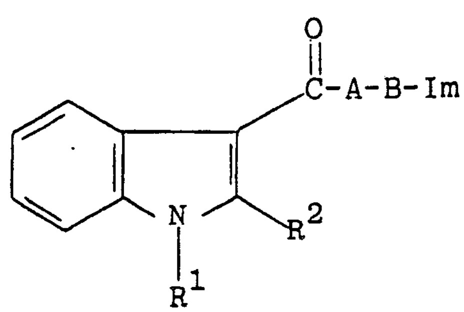

(I)

wherein Im represents an imidazolyl group of formula:

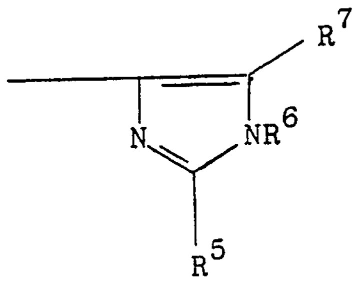

or

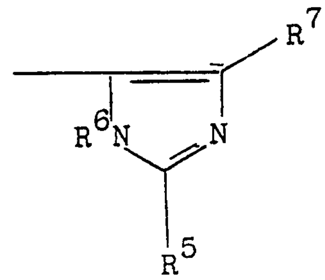

$R^1$ represents a hydrogen atom or a $C_{1-6}$ alkyl, $C_{3-6}$ alkenyl, $C_{3-10}$ alkynyl, $C_{3-7}$ cycloalkyl, $C_{3-7}$ cycloalkyl$C_{1-4}$alkyl, phenyl or phenyl$C_{1-3}$alkyl group;
$R_2$ represents a hydrogen atom or a $C_{1-6}$ alkyl, $C_{3-6}$ alkenyl, $C_{3-7}$ cycloalkyl, phenyl or phenyl$C_{1-3}$alkyl group;
A-B represents the group $R^3R^4C\text{-}CH_2$ or $R^3C=CH$;
$R^3$ and $R^4$, which may be the same or different, each represents a hydrogen atom or a $C_{1-6}$ alkyl group;
one of the groups represented by $R^5$, $R^6$ and $R^7$ represents a hydrogen atom or a $C_{1-6}$ alkyl, $C_{3-7}$ cycloalkyl, $C_{3-6}$ alkenyl, phenyl or phenyl$C_{1-3}$alkyl group, and each of the other two groups, which may be the same or different, represents a hydrogen atom or a $C_{1-6}$ alkyl group;
and physiologically acceptable salts and solvates thereof.

**2.** Compounds as claimed in claim 1 in which $R^1$ represents a hydrogen atom or a $C_{1-3}$ alkyl, $C_{3-4}$ alkenyl, $C_{3-4}$ alkynyl, $C_{5-6}$ cycloalkyl, $C_{5-6}$ cycloalkylmethyl or benzyl group.

**3.** Compounds as claimed in claim 1 or 2 in which $R^2$ represents a hydrogen atom or a $C_{1-3}$ alkyl or phenyl group.

**4.** Compounds as claimed in any of claims 1 to 3 in which A-B represents $CH=CH$ or $R^3R^4C\text{-}CH_2$ where $R^3$ and $R^4$ each independently represent a hydrogen atom or a $C_{1-3}$ alkyl group.

**5.** Compounds as claimed in any of claims 1 to 4 in which $R^5$ and $R^7$ each represent a hydrogen atom or a $C_{1-3}$ alkyl group, and $R^6$ represents a hydrogen atom or a $C_{1-3}$ alkyl, $C_{3-4}$ alkenyl or benzyl group.

**6.** Compounds as claimed in claim 1 in which $R^1$ represents a hydrogen atom or a $C_{1-3}$ alkyl, $C_{3-4}$ alkenyl, $C_{3-4}$ alkynyl,$C_{5-6}$ cycloalkyl or benzyl group; $R^2$ represents a hydrogen atom or a $C_{1-3}$ alkyl group; A-B represents $CH=CH$ or $R^3R^4C\text{-}CH_2$ where $R^3$ and $R^4$ each independently represent a hydrogen atom or a $C_{1-3}$ alkyl group; and $R^5$, $R^6$ and $R^7$ each independently represent a hydrogen atom or a $C_{1-3}$ alkyl group.

**7.** Compounds as claimed in claim 1 in which $R^1$ represents a hydrogen atom or a methyl;prop-2-enyl or cyclopentyl group; $R^2$ represents a hydrogen atom or a methyl group; A-B represents $CR^3R^4\text{-}CH_2$ where $R^3$ and $R^4$ each independently represent a hydrogen atom or a methyl group; $R^5$ and $R^6$ each represent a hydrogen atom; and $R^7$ represents a hydrogen atom or a $C_{1-3}$ alkyl group.

**8.** Compounds as claimed in claim 1 in which A-B represents $R^3R^4C\text{-}CH_2$ and $R^1$, $R^2$, $R^3$, $R^4$, $R^5$, $R^6$ and $R^7$ are as defined in claim 1.

**9.** A compound as claimed in claim 1 selected from: 3-(5-methyl-1H-imidazol-4-yl)-1-(1-methyl-1H-indol-3-yl)-1-propanone;
2-methyl-3-(5-methyl-1H-imidazol-4-yl)-1-(1-methyl-1H-indol-3-yl)-1-propanone;
3-(5-methyl-1H-imidazol-4-yl)-1-(1,2-dimethyl-1H-indol-3-yl) -1-propanone;
1-(1-methyl-1H-indol-3-yl)-3-(5-propyl-1H-imidazol-4-yl)-1-propanone;
2,2-dimethyl-3-(5-methyl-1H-imidazol-4-yl)-1-(1-methyl-1H-indol-3-yl)-1-propanone;
and physiologically acceptable salts and solvates thereof.

**10.** A process for the preparation of a compound of general formula (I) as defined in any of claims 1 to 9 or a physiologically acceptable salt or solvate thereof, which comprises:

(A) reacting a compound of general formula (II)

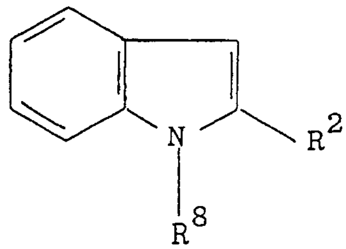

(II)

in which $R^8$ represents a group $R^1$ as defined in claim 1 or a group MgHal, where Hal is a halide ion, or a protected derivative thereof with an acylating reagent derived from an acid of general formula (III)

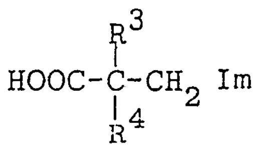

(III)

or a salt or protected derivative thereof, followed if necessary by removal of any protecting group present; or

(B) for the preparation of a compound of formula (I) in which A-B represents the group $R^3C=CH$, condensing a compound of formula (VII)

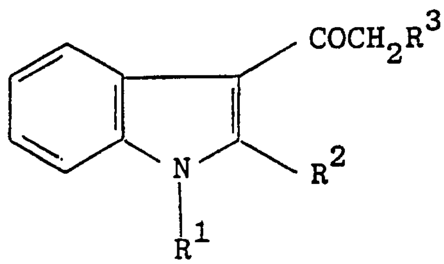

(VII)

or a protected derivative thereof, with a compound of formula (IV)

OHC-Im (IV)

or a protected derivative thereof in the presence of a base, followed if necessary by removal of any protecting groups present; or

(C) converting a compound of formula (I) or a salt or protected derivative thereof into another compound of formula (I) using conventional techniques; or

(D) for the preparation of a compound of formula (I) in which A-B represents the group $R^3R^4C\text{-}CH_2$, oxidising a compound of formula (IX)

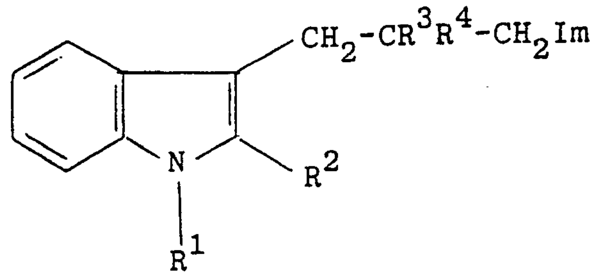

(IX)

or a protected derivative thereof, followed if necessary by removal of any protecting groups present; or

(E) for the preparation of a compound of formula (I) in which A-B represents the group $R^3R^4C-CH_2$, reacting a compound of formula (X)

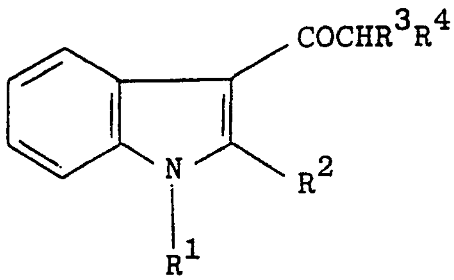

(X)

or a protected derivative thereof, with a compound of formula (XI)

$LCH_2$-IM (XI)

where L represents a leaving atom or group, or a protected derivative thereof, in the presence of base, followed if necessary by removal of any protecting groups present;

(F) removing protecting group(s) from a protected form of a compound of formula (I);

and, when the compound of formula (I) is obtained as a mixture of enantiomers, optionally resolving the mixture to obtain the desired enantiomer;

and/or where the compound of formula (I) is in the form of a free base, optionally converting the free base into a salt.

**11.** A pharmaceutical composition comprising at least one compound of general formula (I) as defined in claim 1 or a physiologically acceptable salt or solvate thereof together with at least one physiologically acceptable carrier or excipient.

**12.** Compounds as claimed in claim 1 in which $R^1$ and $R^2$ each independently represent a hydrogen atom or a $C_{1-6}$ alkyl, $C_{3-6}$ alkenyl, $C_{3-7}$ cycloalkyl, phenyl or phenyl $C_{1-3}$ alkyl group; one of the groups represented by $R^5$, $R^6$ and $R^7$ is a hydrogen atom or a $C_{1-6}$ alkyl, $C_{3-7}$ cycloalkyl, $C_{3-6}$ alkenyl or phenyl $C_{1-3}$ alkyl group, and each of the other two groups, which may be the same or different, represents a hydrogen atom or a $C_{1-6}$ alkyl group; and A-B, $R^3$ and $R^4$ are as defined in claim 1.

**13.** A compound of formula (I) as defined in any of claims 1 to 9 or 12 or a physiologically acceptable salt or solvate thereof for use as an active therapeutic agent.

**Revendications**

**1.** Composés de formule générale (I) :

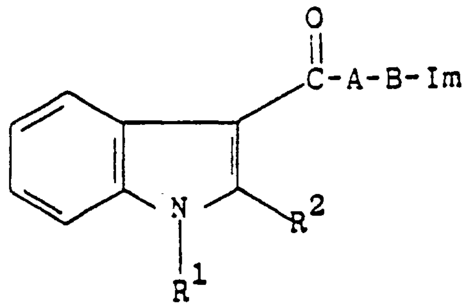

(I)

dans laquelle Im représente un groupe imidazolyle de formule :

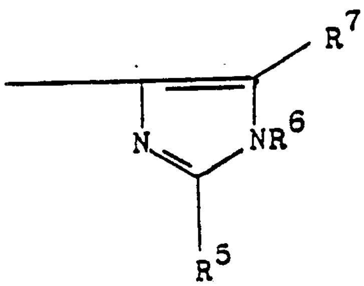

ou

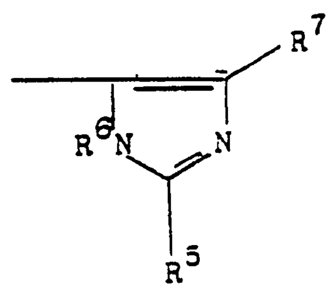

$R^1$ représente un atome d'hydrogène ou un groupe alkyle en $C_1$-$C_6$, alcényle en $C_3$-$C_6$, alcynyle en $C_3$-$C_{10}$, cycloalkyle en $C_3$-$C_7$, cycloalkyl(en $C_3$-$C_7$)alkyle en $C_1$-$C_4$, phényle ou phényl-(alkyle en $C_1$-$C_3$);
$R^2$ représente un atome d'hydrogène ou un groupe alkyle en $C_1$-$C_6$, alcényle en $C_3$-$C_6$, cycloalkyle en $C_3$-$C_7$, phényle ou phényl-(alkyle en $C_1$-$C_3$);
A-B représente le groupe $R^3R^4C$-$CH_2$ ou $R^3C=CH$;
$R^3$ et $R^4$, qui peuvent être identiques ou différents, représentent chacun un atome d'hydrogène ou un groupe alkyle en $C_1$-$C_6$;
l'un des groupes représentés par $R^5$, $R^6$ et $R^7$ est un atome d'hydrogène ou un groupe alkyle en $C_1$-$C_6$, cycloalkyle en $C_3$-$C_7$, alcényle en $C_3$-$C_6$, phényle ou phényl-(alkyle en $C_1$-$C_3$), et chacun des deux autres groupes, qui peuvent être identiques ou différents, représente un atome d'hydrogène ou un groupe alkyle en $C_1$-$C_6$; et leurs sels et solvates physiologiquement acceptables.

2. Composés selon la revendication 1, dans lesquels $R^1$ représente un atome d'hydrogène ou un groupe alkyle en $C_1$-$C_3$, alcényle en $C_3$-$C_4$, alcynyle en $C_3$-$C_4$, cycloalkyle en $C_5$-$C_6$, cycloalkyl(en $C_5$-$C_6$)-méthyle ou benzyle.

3. Composés selon la revendication 1 ou 2, dans lesquels $R^2$ représente un atome d'hydrogène ou un groupe alkyle en $C_1$-$C_3$ ou phényle.

4. Composés selon l'une quelconque des revendications 1 à 3, dans lesquels A-B représente $CH=CH$ ou $R^3R^4C$-$CH_2$, où $R^3$ et $R^4$ représentent chacun indépendamment un atome d'hydrogène ou un groupe alkyle en $C_1$-$C_3$.

5. Composés selon l'une quelconque des revendications 1 à 4, dans lesquels $R^5$ et $R^7$ représentent chacun un atome d'hydrogène ou un groupe alkyle en $C_1$-$C_3$, et $R^6$ représente un atome d'hydrogène ou un groupe alkyle en $C_1$-$C_3$, alcényle en $C_3$-$C_4$ ou benzyle.

6. Composés selon la revendication 1, dans lesquels $R^1$ représente un atome d'hydrogène ou un groupe alkyle en $C_1$-$C_3$, alcényle en $C_3$-$C_4$, alcynyle en $C_3$-$C_4$, cycloalkyle en $C_5$-$C_6$ ou benzyle; $R^2$ représente un atome d'hydrogène ou un groupe alkyle en $C_1$-$C_3$; A-B représente $CH=CH$ ou $R^3R^4C$-$CH_2$, où $R^3$ et $R^4$ représentent chacun indépendamment un atome d'hydrogène ou un groupe alkyle en $C_1$-$C_3$; et $R^5$, $R^6$ et $R^7$ représentent chacun indépendamment un atome d'hydrogène ou un groupe alkyle en $C_1$-$C_3$.

7. Composés selon la revendication 1, dans lesquels $R^1$ représente un atome d'hydrogène ou un groupe méthyle, prop-2-ényle ou cyclopentyle; $R^2$ représente un atome d'hydrogène ou un groupe méthyle; A-B représente $CR^3R^4$-$CH_2$, où $R^3$ et $R^4$ représentent chacun indépendamment un atome d'hydrogène ou un groupe méthyle; $R^5$ et $R^6$ représentent chacun un atome d'hydrogène; et $R^7$ représente un atome d'hydrogène ou un groupe alkyle en $C_1$-$C_3$.

8. Composés selon la revendication 1, dans lesquels A-B représente $R^3R^4C$-$CH_2$ et $R^1$, $R^2$, $R^3$, $R^4$, $R^5$, $R^6$ et $R^7$ sont tels que définis dans la revendication 1.

9. Composé selon la revendication 1, choisi parmi : la 3-(5-méthyl-1H-imidazole-4-yl)-1-(1-méthyl-1H-indole-3-yl)-1-propanone;
la 2-méthyl-3-(5-méthyl-1H-imidazole-4-yl)-1-(1-méthyl-1H-indole-3-yl)-1-propanone;
la 3-(5-méthyl-1H-imidazole-4-yl)-1-(1,2-diméthyl-1H-indole-3-yl)-1-propanone;
la 1-(1-méthyl-1H-indole-3-yl)-3-(5-propyl-1H-imidazole-4-yl)-1-propanone;
la 2,2-diméthyl-3-(5-méthyl-1H-imidazole-4-yl)-1-(1-méthyl-1H-indole-3-yl)-1-propanone;
et leurs sels et solvates physiologiquement acceptables.

10. Procédé de préparation d'un composé de formule générale (I) telle que définie dans l'une quelconque des revendications 1 à 9 ou d'un de ses sels ou solvates physiologiquement acceptables, qui comprend les étapes qui consistent :
(A) à faire réagir un composé de formule générale (II)

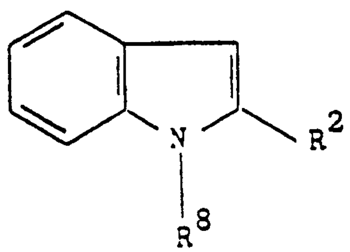

(II)

dans laquelle $R^8$ représente un groupe $R^1$ tel que défini dans la revendication 1 ou un groupe MgHal, où Hal est un ion halogénure, ou un de ses dérivés protégés, avec un réactif d'acylation dérivé d'un acide de formule générale (III) :

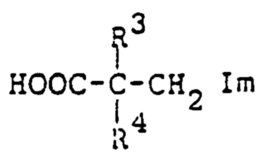

(III)

ou un de ses sels ou dérivés protégés, puis, le cas échéant, à éliminer les groupes protecteurs éventuellement présents; ou
(B) pour la préparation d'un composé de formule (I), dans laquelle A-B représente le groupe $R^3C=CH$, à condenser un composé de formule (VII)

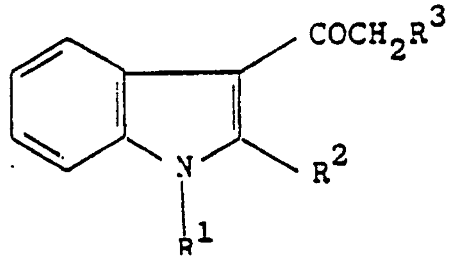

(VII)

ou un de ses dérivés protégés, avec un composé de formule (IV)

OHC-Im (IV)

ou un de ses dérivés protégés, en présence d'une base, puis éventuellement à éliminer les groupes protecteurs éventuellement présents; ou

(C) à transformer un composé de formule (I) ou un de ses sels ou dérivés protégés en un autre composé de formule (I) en utilisant des techniques classiques; ou

(D) pour la préparation d'un composé de formule (I), dans laquelle A-B représente le groupe $R^3R^4C$-$CH_2$, à oxyder un composé de formule (IX)

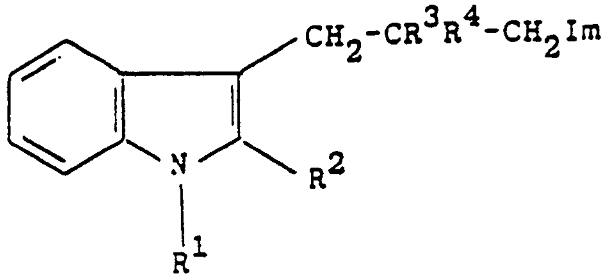

(IX)

ou un de ses dérivés protégés, puis, le cas échéant, à éliminer les groupes protecteurs éventuellement présents; ou

(E) pour la préparation d'un composé de formule (I), dans laquelle A-B représente le groupe $R^3R^4C$-$CH_2$, à faire réagir un composé de formule (X)

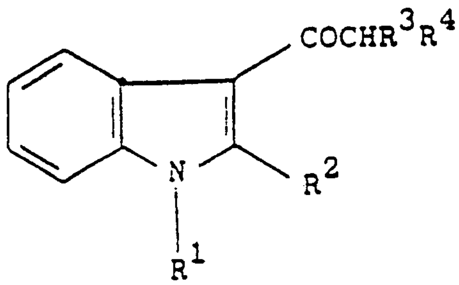

(X)

ou un de ses dérivés protégés, avec un composé de formule (XI)

$LCH_2$-Im (XI)

dans laquelle L représente un atome ou un groupe partant, ou un de ses dérivés protégés, en présence d'une base, puis, le cas échéant, à éliminer les groupes protecteurs éventuellement présents;

(F) à éliminer le(s) groupe(s) protecteur(s) d'une forme protégée d'un composé de formule (I); puis, si l'on obtient le composé de formule (I) sous forme d'un mélange d'énantiomères, éventuellement à résoudre le mélange pour obtenir l'énantiomère recherché;

et/ou, si le composé de formule (I) est sous la forme d'une base libre, à transformer éventuellement la base libre en un sel.

**11.** Composition pharmaceutique comprenant au moins un composé de formule générale (I) telle que définie dans la revendication 1 ou un de ses sels ou solvates physiologiquement acceptables, ainsi qu'au moins un véhicule ou un excipient physiologiquement acceptable.

**12.** Composés selon la revendication 1, dans lesquels chaque $R^1$ et $R^2$ représente indépendamment un atome d'hydrogène ou un groupe alkyle en $C_1$-$C_6$, alcényle en $C_3$-$C_6$, cycloalkyle en $C_3$-$C_7$, phényle ou phényl(alkyle en $C_1$-$C_3$); l'un des groupes représentés par $R^5$, $R^6$ et $R^7$ est un atome d'hydrogène ou un groupe alkyle en $C_1$-$C_6$, cycloalkyle en $C_3$-$C_7$, alcényle en $C_3$-$C_6$ ou phényl(alkyle en $C_1$-$C_3$), et

chacun des deux atures groupes, qui peuvent être identiques ou différents, représente un atome d'hydrogène ou un groupe alkyle en $C_1$-$C_6$; et A-B, $R^3$ et $R^4$ sont tels que définis dans la revendication 1.

13. Composé de formule (I) telle que définie dans l'une quelconque des revendications 1 à 9 ou 12, ou sel ou solvate physiologiquement acceptable de celui-ci, utilisable comme agent thérapeutiquement actif.

**Patentansprüche**

1. Verbindungen der allgemeinen Formel (I):

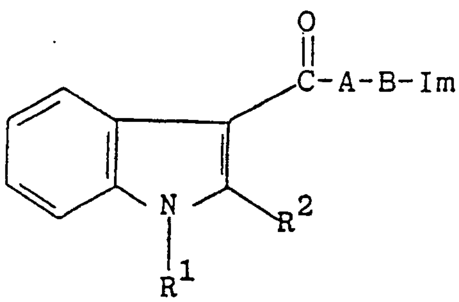

(I)

worin Im eine Imidazolylgruppe der Formel:

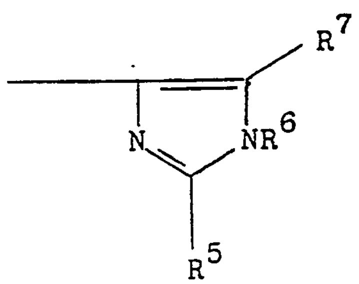

oder

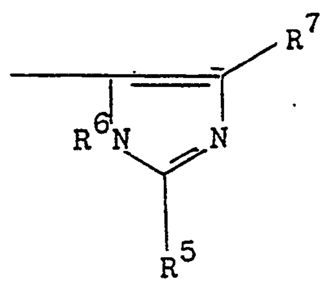

bedeutet; $R^1$ ein Wasserstoffatom oder eine $C_{1-6}$-Alykl-,$C_{3-6}$ -Alkenyl-, $C_{3-10}$-Alkinyl-, $C_{3-7}$-Cycloalkyl-, $C_{3-7}$-Cycloalkyl-$C_{1-4}$ -alkyl-, Phenyl- oder Phenyl-$C_{1-3}$-alkylgruppe bedeutet;
$R^2$ ein Wasserstoffatom oder eine $C_{1-6}$-Alkyl-, $C_{3-6}$-Alkenyl-, $C_{3-7}$-Cycloalkyl-, Phenyl- oder Phenyl-$C_{1-3}$-alkylgruppe bedeutet;
A-B die Gruppe $R^3R^4C$-$CH_2$ oder $R^3$ C=CH bedeutet;
$R^3$ und $R^4$, die gleich oder unterschiedlich sein können, je ein Wasserstoffatom oder eine $C_{1-6}$ -Alkylgruppe bedeuten;
eine der Gruppen, die durch $R^5$, $R^6$ und $R^7$ dargestellt wird, ein Wasserstoffatom oder eine $C_{1-6}$ -Alkyl-, $C_{3-7}$-Cycloalkyl-, $C_{3-6}$ -Alkenyl-, Phenyl- oder Phenyl-$C_{1-3}$ -alkylgruppe bedeutet und jede der beiden anderen Gruppen, die gleich oder unterschiedlich sein können, ein Wasserstoffatom oder eine $C_{1-6}$ Alkylgruppe bedeutet;
und ihre physiologisch annehmbaren Salze und Solvate.

2. Verbindungen nach Anspruch 1, dadurch **gekennzeichnet,** daß $R^1$ ein Wasserstoffatom oder eine $C_{1-3}$-Alkyl-, $C_{3-4}$-Alkenyl-, $C_{3-4}$-Alkinyl-, $C_{5-6}$-Cycloalkyl-, $C_{5-6}$-Cycloalkylmethyl- oder Benzylgruppe bedeutet.

3. Verbindungen nach Anspruch 1 oder 2, dadurch **gekennzeichnet**, daß $R^2$ ein Wasserstoffatom oder eine $C_{1-3}$ -Alkyl- oder Phenylgruppe bedeutet.

4. Verbindungen nach einem der Ansprüche 1 bis 3, dadurch **gekennzeichnet,** daß A-B CH=CH oder $R^3R^4C$-$CH_2$ bedeutet, worin $R^3$ und $R^4$ je unabhängig ein Wasserstoffatom oder eine $C_{1-3}$ -Alkylgruppe bedeuten.

5. Verbindungen nach einem der Ansprüche 1 bis 4, dadurch **gekennzeichnet,** daß $R^5$ und $R^7$ je ein Wasserstoffatom oder eine $C_{1-3}$ -Alkylgruppe bedeuten und $R^6$ ein Wasserstoffatom oder eine $C_{1-3}$ -Alkyl-, $C_{3-4}$ -Alkenyl- oder Benzyl-gruppe bedeutet.

6. Verbindungen nach Anspruch 1, dadurch **gekennzeichnet,** daß $R^1$ ein Wasserstoffatom oder eine $C_{1-3}$-Alkyl-, $C_{3-4}$-Alkenyl-, $C_{3-4}$-Alkinyl-, $C_{5-6}$ -Cycloalkyl- oder Benzylgruppe bedeutet; $R^2$ ein Wasserstoffatom oder eine $C_{1-3}$ -Alkylgruppe bedeutet; A-B CH=CH oder $R^3R^4C\text{-}CH_2$ bedeutet,worin $R^3$ und $R^4$ je unabhängig ein Wasserstoffatom oder eine $C_{1-3}$ -Alkylgruppe bedeuten; und $R^5$, $R^6$ und $R^7$ je unabhängig ein Wasserstoffatom oder eine $C_{1-3}$ -Alkylgruppe bedeuten.

7. Verbindungen nach Anspruch 1, dadurch **gekennzeichnet,** daß $R^1$ ein Wasserstoffatom oder eine Methyl-, Prop-2-enyl- oder Cyclopentylgruppe bedeutet; $R^2$ ein Wasserstoffatom oder eine Methylgruppe bedeutet; A-B $CR^3R^4\text{-}CH_2$ bedeutet, worin $R^3$ und $R^4$ je unabhängig ein Wasserstoffatom oder eine Methylgruppe bedeuten; $R^5$ und $R^6$ je ein Wasserstoffatom bedeuten; und $R^7$ ein Wasserstoffatom oder eine $C_{1-3}$ -Alkylgruppe bedeutet.

8. Verbindungen nach Anspruch 1, dadurch **gekennzeichnet,** daß A-B $R^3$ $R^4$ $C\text{-}CH_2$ bedeutet und $R^1$, $R^2$, $R^3$, $R^4$, $R^5$, $R^6$ und $R^7$ die in Anspruch 1 gegebenen Definitionen besitzen.

9. Verbindung nach Anspruch 1, ausgewählt unter:
3-(5-Methyl-1H-imidazol-4-yl)-1-(1-methyl-1H-indol-3-yl)-1-propanon;
2-Methyl-3-(5-methyl-1H-imidazol-4-yl)-1-(1-methyl-1H-indol-3-yl)-1-propanon;
3-(5-Methyl-1H-imidazol-4-yl)-1-(1,2-dimethyl-1H-indol-3-yl)-1-propanon;
1-(1-Methyl-1H-indol-3-yl)-3-(5-propyl-1H-imidazol-4-yl)-1-propanon;
2,2-Dimethyl-3-(5-methyl-1H-imidazol-4-yl)-1-(1-methyl-1H-indol-3-yl)-1-propanon;
und ihre physiologisch annehmbaren Salze und Solvate.

10. Verfahren zur Herstellung einer Verbindung der allgemeinen Formel (I) nach einem der Ansprüche 1 bis 9 oder eines physiologisch annehmbaren Salzes oder Solvats davon, dadurch **gekennzeichnet,** daß
(A) eine Verbindung der allgemeinen Formel (II):

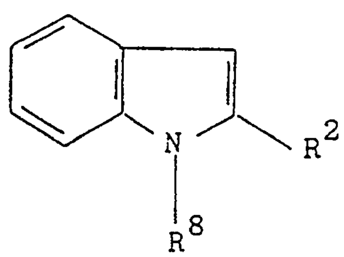

(II)

worin $R^8$ eine Gruppe $R^1$ , wie sie in Anspruch 1 definiert wurde, oder eine Gruppe MgHal bedeutet, worin Hal für ein Halogenidion steht, oder ein geschütztes Derivat davon mit einem Acylierungsreagens, das sich von einer Säure der allgemeinen Formel (III):

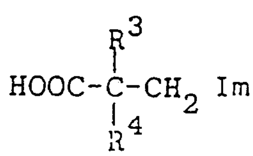

(III)

ableitet, oder mit einem Salz oder geschützten Derivat davon umgesetzt wird und gegebenenfalls anschließend irgendwelche vorhandenen Schutzgruppen entfernt werden; oder
(B) für die Herstellung einer Verbindung der Formel (I), worin A-B die Gruppe $R^3C=CH$ bedeutet, eine Verbindung der Formel (VII):

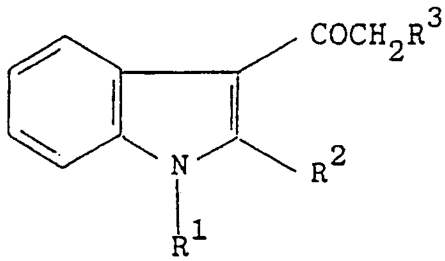

(VII)

oder ein geschütztes Derivat davon mit einer Verbindung der Formel (IV):

OHC-Im (IV)

oder einem geschützten Derivat davon in Anwesenheit einer Base kondensiert wird und anschließend gegebenenfalls irgendwelche vorhandenen Schutzgruppen entfernt werden; oder
(C) eine Verbindung der Formel (I) oder ein Salz oder ein geschütztes Derivat davon in eine andere Verbindung der Formel (I) unter Verwendung an sich bekannter Verfahren überführt wird; oder
(D) für die Herstellung einer Verbindung der Formel (I), worin A-B die Gruppe $R^3R^4C-CH_2$ bedeutet, eine Verbindung der Formel (IX):

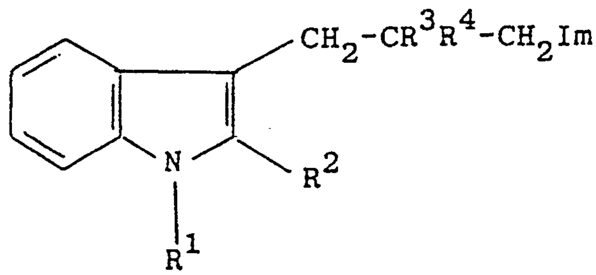

(IX)

oder ein geschütztes Derivat davon oxidiert wird und anschließend gegebenenfalls irgendwelche vorhandenen Schutzgruppen entfernt werden; oder
(E) für die Herstellung einer Verbindung der Formel (I), worin A-B die Gruppe $R^3R^4C-CH_2$ bedeutet, eine Verbindung der Formel (X):

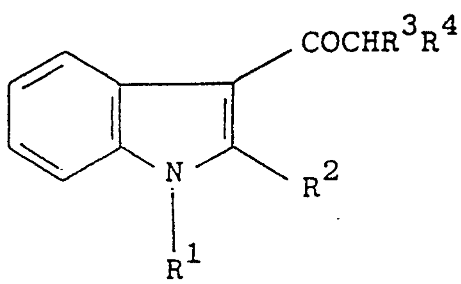

(X)

oder ein geschütztes Derivat davon mit einer Verbindung der Formel (XI):

$LCH_2$-Im (XI)

worin L ein abspaltbares Atom oder eine abspaltbare Gruppe bzw. ein austretendes Atom oder eine austretende Gruppe bedeutet, oder mit einem geschützten Derivat davon in Anwesenheit einer Base umgesetzt wird und anschließend gegebenenfalls irgendwelche vorhandenen Schutzgruppen entfernt werden;
(F) die Schutzgruppe(n) von der geschützten Form der Verbindung (I) entfernt wird bzw. werden und, wenn die erhaltene Verbindung der Formel (I) ein Gemisch der Enantiomeren ist, gegebenenfalls das Gemisch unter Herstellung der gewünschten Enantiomeren gespalten wird;

und/oder, wenn die Verbindung der Formel (I) in Form der freien Base vorliegt, gegebenenfalls die freie Base in das Salz überführt wird.

**11.** Pharmazeutisches Präparat, dadurch **gekennzeichnet**, daß es mindestens eine Verbindung der allgemeinen Formel (I) nach Anspruch 1 oder ein physiologisch annehmbares Salz oder Solvat davon zusammen mit mindestens einem physiologisch annehmbaren Träger oder Verdünnungsmittel enthält.

**12.** Verbindungen nach Anspruch 1, dadurch **gekennzeichnet**, daß $R^1$ und $R^2$ je unabhängig ein Wasserstoffatom oder eine $C_{1-6}$ -Alkyl-, $C_{3-6}$-Alkenyl-, $C_{3-7}$-Cyclo-alkyl-, Phenyl- oder Phenyl-$C_{1-3}$-alkylgruppe bedeuten; eine der Gruppen, die durch $R^5$, $R^6$ und $R^7$ dargestellt wird, ein Wasserstoffatom oder eine $C_{1-6}$ -Alkyl-, $C_{3-7}$-Cycloalkyl-, $C_{3-6}$ -Alkenyl- oder Phenyl-$C_{1-3}$ -alkylgruppe bedeutet und jede der beiden anderen Gruppen, die gleich oder unterschiedlich sein können, ein Wasserstoffatom oder eine $C_{1-6}$ -Alkylgruppe bedeutet; und A-B, $R^3$ und $R^4$ die in Anspruch 1 gegebenen Bedeutungen besitzen.

**13.** Verbindung der Formel (I) nach einem der Ansprüche 1 bis 9 oder 12 oder ein physiologisch annehmbares Salz oder Solvat davon für die Verwendung als aktives therapeutisches Mittel.